(19) Europäisches Patentamt
European Patent Office
Office européen des brevets

(11) Numéro de publication : **0 145 513**
**B1**

(12) # FASCICULE DE BREVET EUROPÉEN

(45) Date de publication du fascicule du brevet : **23.03.88**

(21) Numéro de dépôt : **84401758.2**

(22) Date de dépôt : **04.09.84**

(51) Int. Cl.⁴ : **C 07 C 87/60**, C 07 C 87/62, C 07 C 91/06, C 07 C 91/10, A 61 K 7/13

(54) **Nouveaux dérivés diméthylés de nitro-3 amino-4 aniline, leur procédé de préparation et leur utilisation en teinture des fibres kératiniques.**

(30) Priorité : **08.09.83 FR 8314337**
**23.08.84 FR 8413147**

(43) Date de publication de la demande :
**19.06.85 Bulletin 85/25**

(45) Mention de la délivrance du brevet :
**23.03.88 Bulletin 88/12**

(84) Etats contractants désignés :
**AT BE CH DE FR GB IT LI NL SE**

(56) Documents cités :
**EP-A- 0 075 242**
**FR-A- 1 449 648**
**GB-A- 2 086 408**
**US-A- 3 973 900**
**Le dossier contient des informations techniques présentées postérieurement au dépôt de la demande et ne figurant pas dans le présent fascicule.**

(73) Titulaire : **L'OREAL**
**14, Rue Royale**
**F-75008 Paris (FR)**

(72) Inventeur : **Bugaut, Andrée**
**15 Rue Anna Jacquin**
**F-92100 Boulogne-Billancourt (FR)**
Inventeur : **Genet, Alain**
**82 Boulevard Fichot**
**F-93360 Neuilly-Plaisance (FR)**
Inventeur : **Cotteret, Jean**
**6 Allée des Cerisiers Les Hauts de Limay**
**F-78520 Limay (FR)**
Inventeur : **Junino, Alex**
**16, Rue du Docteur Bergonié**
**F-93190 Livry-Gargan (FR)**

(74) Mandataire : **Casalonga, Axel et al**
**BUREAU D.A. CASALONGA OFFICE JOSSE & PETIT**
**Morassistrasse 8**
**D-8000 München 5 (DE)**

**EP 0 145 513 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à de nouveaux dérivés diméthylés de nitro-3 amino-4 aniline, à leur procédé de préparation et à leur utilisation en teinture des fibres kératiniques, et en particulier des cheveux humains.

Il est bien connu que pour conférer aux cheveux une coloration directe ou des reflets complémentaires dans le cas de la coloration d'oxydation, on peut utiliser des dérivés nitrés de la série benzénique. On a déjà préconisé l'utilisation de nitroparaphénylènediamines telles que plus particulièrement la nitroparaphénylènediamine et la méthyl-2 nitro-5 paraphénylènediamine tant en teinture directe qu'en teinture d'oxydation.

La parfaite innocuité de ces nitroparaphénylènediamines a cependant été mise en doute ces dernières années et on a cherché de ce fait à remplacer ces colorants dans les compositions tinctoriales pour cheveux.

Par ailleurs, on a constaté d'une manière générale une mauvaise conservation des nitroparaphénylènediamines dans le milieu alcalin réducteur, comprenant notamment du bisulfite de sodium, utilisé habituellement dans les compositions de teinture d'oxydation. On a donc recherché également des nitroparaphénylènediamines présentant une bonne conservation en milieu alcalin réducteur.

La demande de brevet GB-A-2 086 408 décrit ainsi des méthyl-2 amino-4 nitro-5 anilines N-substituées satisfaisantes du point de vue innocuité et stabilité en milieu alcalin réducteur.

En poursuivant ses recherches, la demanderesse a découvert de nouveaux dérivés diméthylés de nitro-3 amino-4 aniline présentant à la fois une très faible toxicité et une très bonne stabilité en solution, notamment lorsqu'ils sont utilisés dans une composition tinctoriale d'oxydation en présence d'un agent alcalin et d'un réducteur tel que le bisulfite de sodium.

La demanderesse a constaté également que les teintures capillaires obtenues à l'aide de ces nouveaux composés présentent une bonne stabilité aux intempéries, à la lumière et au lavage.

La présente invention a donc pour objet de nouveaux dérivés diméthylés de nitro-3 amino-4 aniline ainsi que leur procédé de préparation.

L'invention vise également des compositions tinctoriales pour fibres kératiniques, et en particulier pour cheveux humains, contenant ces dérivés diméthylés de nitro-3 amino-4 aniline, compositions tinctoriales qui peuvent être utilisées aussi bien pour la coloration directe des cheveux que pour la coloration par voie d'oxydation.

D'autres objets et avantages de la présente invention apparaîtront à la lecture de la description et des exemples qui suivent.

Les dérivés diméthylés de nitro-3 amino-4 aniline selon l'invention répondent à la formule (I) suivante :

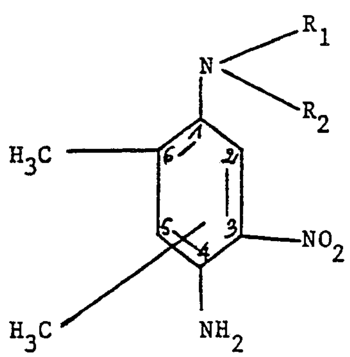

(I)

dans laquelle le radical $CH_3$ à position variable peut se trouver en position 2 ou 5 du cycle benzénique et $R_1$ et $R_2$ désignent indépendamment l'un de l'autre un atome d'hydrogène, un groupe alkyle inférieur, mono- ou polyhydroxyalkyle inférieur ou aminoalkyle inférieur dont le groupement amino peut être éventuellement mono- ou disubstitué par un radical alkyle inférieur ou par un radical mono- ou polyhydroxyalkyle inférieur, étant entendu que les deux symboles $R_1$ et $R_2$ ne peuvent être simultanément différents de l'hydrogène que lorsque le radical $CH_3$ mobile se trouve en position 5.

Ces dérivés de formule (I) peuvent aussi exister sous forme de sels cosmétiquement acceptables.

Selon l'invention, le groupe alkyle inférieur est un groupe alkyle contenant 1 à 6 atomes de carbone et de préférence 1 à 4 atomes de carbone.

Des radicaux $R_1$ et $R_2$ particulièrement préférés selon l'invention sont les radicaux méthyle, éthyle, n-propyle, β-hydroxyéthyle, β-hydroxypropyle, γ-hydroxypropyle, β,γ-dihydroxypropyle, β-aminoéthyle, γ-aminopropyle et β-N,N-diéthylaminoéthyle.

Des composés préférés sont ceux dans lesquels l'un au moins des radicaux $R_1$ et $R_2$ désigne un atome d'hydrogène.

Des composés particulièrement inoffensifs et solides au lavage et à la lumière sont les composés suivants :

— diméthyl-2,6 nitro-3 paraphénylènediamine
— diméthyl-5,6 nitro-3 paraphénylènediamine
— amino-4 diméthyl-2,6 nitro-3 N-méthylaniline
— amino-4 diméthyl-5,6 nitro-3 N-β-hydroxyéthylaniline
— amino-4 diméthyl-5,6 nitro-3 N-β,γ-dihydroxypropylaniline
— amino-4 diméthyl-5,6 nitro-3 N-β-aminoéthylaniline
— amino-4 diméthyl-5,6 nitro-3 N-(β-diéthylaminoéthyl) aniline.

La diméthyl-2,5 nitro-3 paraphénylènediamine également synthétisée présente des propriétés de colorant mais ne présente pas les avantages importants des composés préférés. Leurs propriétés intéressantes semblent être dues, sans que cette interprétation soit limitative, à la présence d'un radical méthyle en para du groupement $NO_2$.

Les composés de formule (I) dans laquelle $R_1$ et $R_2$ désignent tous deux de l'hydrogène, peuvent être préparés par nitration des composés de formule (II) suivante :

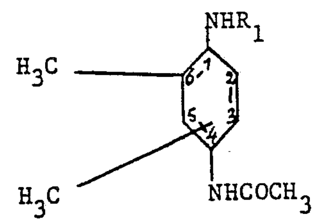

(II)

dans laquelle $R_1$ désigne un atome d'hydrogène lorsque $CH_3$ est en position 2 et $R_1$ désigne le groupe acétyle($-COCH_3$) lorsque $CH_3$ est en position 5.

Lorsque le radical $-CH_3$ est en position 5 avec $R_1$ désignant $-COCH_3$, il s'agit du dérivé N,N'-diacétylé de la diméthyl-5,6 paraphénylène diamine qui est un composé connu.

Lorsque le radical $CH_3$ est en position 2, $R_1$ désignant hydrogène, il s'agit de la diméthyl-2,6 acétylamino-4 aniline qui est un composé nouveau obtenu par acétylation ménagée dans l'eau, à température ambiante, en présence de sulfite de sodium, du dichlorhydrate de diméthyl-2,6 paraphénylènediamine.

La nitration des composés II est effectuée de façon classique. Les composés II, après avoir été mis en solution à température ambiante, dans l'acide sulfurique à 96 %, sont traités entre −5 et 0 °C par un mélange sulfonitrique conduisant aux composés de formule III suivants :

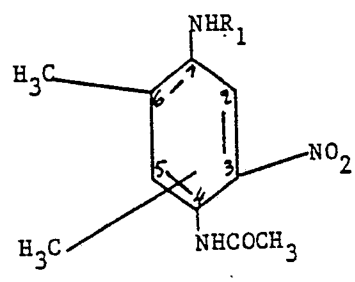

(III)

dans laquelle $R_1$ a les mêmes significations que dans la formule (II) ci-dessus et $CH_3$ les mêmes positions que celles indiquées ci-dessus.

Les composés de formule (III) ainsi obtenus sont ensuite hydrolysés à l'aide d'acide chlorhydrique.

Les composés de formule (I) dans laquelle l'un au moins des symboles $R_1$ et $R_2$ est différent de l'hydrogène, sont préparés selon les deux modes opératoires suivants, selon qu'il s'agit de dérivés de la diméthyl-2,6 nitro-3 paraphénylènediamine ou de dérivés de la diméthyl-5,6 nitro-3 paraphénylènediamine.

1. Préparation des amines secondaires dérivées de la diméthyl-2,6 nitro-3 paraphénylènediamine.

Il est nécessaire dans ce cas de conduire l'alkylation de l'amine en position 1 sur un précurseur qui est le p-toluène sulfonamide de la diméthyl-2,6 nitro-4 aniline. Comme agents alkylants, on peut citer à titre d'exemples non limitatifs, les sulfates d'alkyle ou les halogénures d'alkyle, substitués ou non. Après désulfonation par hydrolyse, la nitro-4 aniline alkylée est réduite en diméthylacétylation de l'amine primaire puis hydrolysée.

Le schéma réactionnel est le suivant :

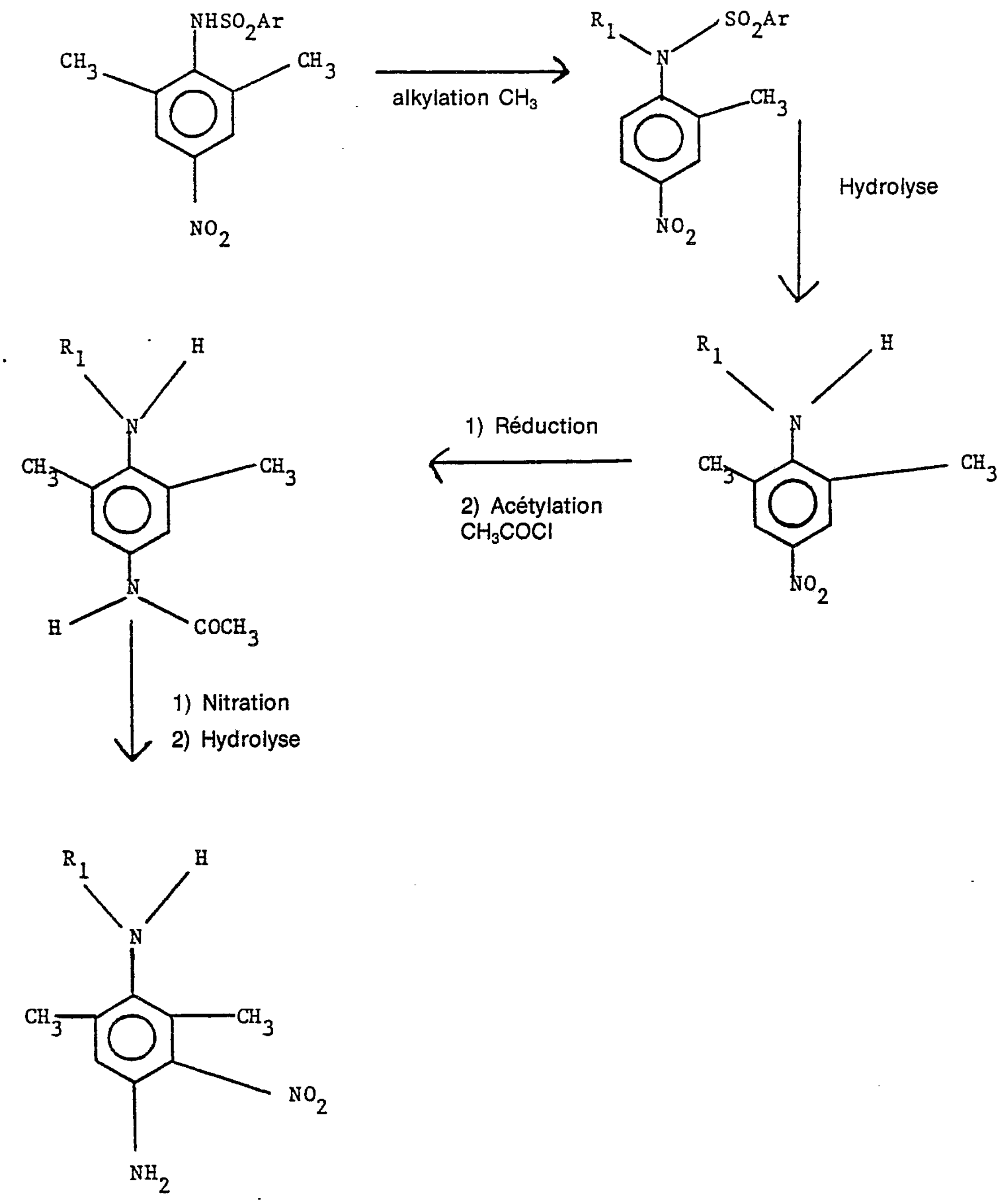


Ar désignant le groupe p-tolyle et $R_1$ ayant les significations indiquées dans la formule (I), à l'exception de l'hydrogène.

2. Préparation des amines secondaires et tertiaires dérivées de la diméthyl-5,6 nitro-3 paraphénylènediamine

Dans ce cas, la substitution de l'amine en 1, plus réactive, peut être conduite directement à partir de la diméthyl-5,6 nitro-3 paraphénylènediamine par alkylation à l'aide d'un halogénure d'alkyle, substitué ou non, d'un époxyde ou par ouverture d'une oxazoline-1,3 one-3 intermédiaire.

Les compositions tinctoriales conformes à l'invention contiennent, dans un milieu solvant, au moins un composé répondant à la formule (I) et peuvent être utilisées pour la coloration directe des fibres kératiniques ou pour la coloration d'oxydation de ces fibres, auquel cas les composés de formule (I) confèrent des reflets complémentaires à la coloration de base obtenue par développement oxydant de précurseurs de colorants d'oxydation.

Ces compositions contiennent les composés selon l'invention dans des proportions comprises entre 0,001 et 5 % en poids et de préférence entre 0,05 et 2 % en poids par rapport au poids total de la composition.

Le milieu solvant est de préférence un véhicule cosmétique généralement constitué par de l'eau mais on peut également ajouter, dans les compositions, des solvants organiques pour solubiliser des composés qui ne seraient pas suffisamment solubles dans l'eau. Parmi ces solvants, on peut citer les alcanols inférieurs tels que l'éthanol et l'isopropanol, les alcools aromatiques comme l'alcool benzylique, les polyols tels que le glycérol, les glycols ou éthers de glycols comme le butoxy-2 éthanol ou l'éthoxy-2 éthanol, l'éthylèneglycol, le propylèneglycol, le monoethyléther et le monométhyléther du diethyleneglycol ainsi que les produits analogues et leurs mélanges. Ces solvants sont de préférence présents dans des proportions allant de 1 à 75% en poids et en particulier de 5 à 50% en poids par rapport au poids total de la composition.

Ces compositions peuvent contenir des agents tensio-actifs anioniques, cationiques, non-anioniques, amphotères ou leurs mélanges. Ces produits tensio-actifs sont présents dans les compositions de l'invention dans des proportions comprises entre 0,5 et 55 % en poids et de préférence entre 4 et 40 % en poids par rapport au poids total de la composition.

Les compositions peuvent être épaissies de préférence avec des composés choisis parmi l'alginate de sodium, la gomme arabique, les dérivés de la cellulose tels que la méthylcellulose, l'hydroxyéthylcellulose, l'hydroxypropylméthylcellulose, la carboxyméthylcellulose et les polymères divers ayant la fonction d'épaississant tels que plus particulièrement les dérivés d'acide acrylique. Il est également possible d'utiliser des agents épaississants minéraux tels que la bentonite. Ces agents épaississants sont présents de préférence dans les proportions comprises entre 0,5 et 10 % en poids et en particulier entre 0,5 et 3 % en poids par rapport au poids total de la composition.

Les compositions selon l'invention peuvent également contenir divers adjuvants habituellement utilisés dans les compositions tinctoriales pour cheveux et en particulier des agents de pénétration, des agents dispersants, des agents séquestrants, des agents filmogènes, des tampons et des parfums.

Ces compositions peuvent se présenter sous des formes diverses telles que liquide, crème, gel ou toute autre forme appropriée pour réaliser une teinture des cheveux. Elles peuvent en outre être conditionnées en flacons aérosols en présence d'un agent propulseur.

Le pH de ces compositions tinctoriales peut être compris entre 3 et 11,5, de préférence entre 5 et 11,5. On l'ajuste à la valeur souhaitée à l'aide d'un agent alcalinisant tel que l'ammoniaque, le carbonate de sodium, de potassium ou d'ammonium, les hydroxydes de sodium ou de potassium, les alcanolamines telles que la mono, la di- ou la triéthanolamine, l'amino-2 méthyl-2 propanol-1, l'amino-2 méthyl-2 propanediol-1,3, les alkylamines telles que l'éthylamine ou la triéthylamine ou à l'aide d'un agent d'acidification tel que les acides phosphorique, chlorhydrique, tartrique, acétique, lactique ou citrique.

Lorsque les compositions sont destinées à être utilisées dans un procédé de coloration directe des cheveux, elles peuvent contenir en plus des composés conformes à l'invention d'autres colorants directs tels que des colorants azoïques ou anthraquinoniques, comme par exemple la tétraamino-1,4,5,8 anthraquinone, des colorants nitrés de la série benzénique différents des composés de formule (I) et plus particulièrement les composés suivants :

— la méthyl-2 nitro-6 aniline,
— le méthyl-2 amino-4 nitro-5 phénol,
— la nitro-3 N'-β-hydroxyéthylamino-4 N-méthylaniline,
— la nitro-3 N'-β-hydroxyéthyl amino-4 N-méthyl, N-β-hydroxyéthylaniline,
— l'amino-4 nitro-3 phénol,
— l'amino-3 nitro-4 phénol,
— l'amino-2 nitro-3 phénol,
— le N-β-hydroxyéthylamino-2 nitro-5 phénol,
— le N-β-hydroxyéthylamino-2 nitro-5 anisole,
— la méthyl-2 nitro-5 N,N'(-β-hydroxyéthyl) paraphénylènediamine,
— la nitro-3 N'-(β-hydroxyéthyl) amino-4 N,N-bis-(β-hydroxyéthyl) aniline,
— la nitro-3 N'-(β-aminoéthyl) amino-4 N,N-bis-(β-hydroxyéthyl) aniline,
— l'amino-4 méthyl-2 nitro-3 N-β-hydroxyéthylaniline,
— le [N-(β-hydroxyéthyl) amino-4 nitro-3] phényl, β-hydroxyéthyl éther,
— le (N-méthylamino-3 nitro-4) phényl, β-hydroxyéthyl éther.
— l'isopropyl-2 nitro-6 aniline,

utilisés sous forme libre ou sous forme de leurs sels.

Les concentrations de ces colorants directs autres que les colorants de formule (I) peuvent être comprises entre 0,001 et 5 % en poids par rapport au poids total de la composition.

Ces compositions, mises en œuvre dans un procédé de teinture par coloration directe, sont appliquées sur les fibres kératiniques pendant un temps de pose variant de 5 à 50 minutes, puis les fibres sont rincées, éventuellement lavées, rincées à nouveau et séchées.

Les compositions selon l'invention peuvent également être mises en œuvre sous forme de lotions capillaires de mise en plis destinées tout à la fois à conférer aux cheveux une légère coloration et à améliorer la tenue de la mise en plis. Dans ce cas, elles se présentent sous forme de solutions aqueuses, alcooliques ou hydroalcooliques renfermant au moins une résine cosmétique et leur application s'effectue sur des cheveux humides préalablement lavés et rincés qui sont éventuellement enroulés puis séchés.

Les résines cosmétiques utilisées dans les lotions de mise en plis peuvent être en particulier la polyvinylpyrrolidone, les copolymères acide crotonique-acétate de vinyle, vinylpyrrolidone-acétate de vinyle, anhydride maléique-éther butylvinylique, anhydride maléique-éther méthylvinylique ainsi que tout autre polymère cationique, anionique, non ionique ou amphotère habituellement utilisé dans ce type de composition. Ces résines cosmétiques entrent dans les compositions de l'invention à raison de 0,5 à 4 % en poids, et de préférence de 1 à 3 % en poids sur la base du poids total de la composition.

Lorsque les compositions selon l'invention constituent des teintures d'oxydation impliquant la révélation par un oxydant, les composés de formule (I) conformes à l'invention sont essentiellement utilisés en vue d'apporter des reflets à la teinture finale.

Ces compositions contiennent alors en association avec au moins un colorant nitré de formule (I) et éventuellement d'autres colorants nitrés tels que ceux mentionnés ci-dessus, des précurseurs de colorants par oxydation.

Elles peuvent contenir par exemple des paraphénylènediamines telles que : la paraphénylènediamine, la paratoluylènediamine, la chloro-2 paraphénylènediamine, la diméthyl-2,6 paraphénylènediamine, la diméthyl-2,6 méthoxy-3 paraphénylènediamine, la N-(β-méthoxyéthyl) paraphénylènediamine, la N,N-(β-hydroxyéthyl) paraphénylènediamine, la N,N-(éthyl, carbamylméthyl) amino-4 aniline, ainsi que leurs sels.

Elles peuvent également contenir des paraaminophénols, par exemple : le paraaminophénol, le N-méthyl paraaminophénol, le chloro-2-amino-4 phényl, le chloro-3 amino-4 phénol, le méthyl-2 amino-4 phénol, et leurs sels.

Elles peuvent également contenir de l'orthoaminophénol.

Elles peuvent aussi contenir des dérivés hétérocycliques, par exemple : la diamino-2,5 pyridine, l'amino-7 benzomorpholine.

Les compositions selon l'invention peuvent contenir en association avec les précurseurs de colorants par oxydation, des coupleurs bien connus dans l'état de la technique.

A titre de coupleurs, on peut citer notamment : les métadiphénols tels que la résorcine, la méthyl-2 résorcine, les métaaminophénols tels que : le métaaminophénol, le méthyl-2 amino-5 phénol, le méthyl-2 N-méthyl-amino-5 phénol, le méthyl-2 N-(β-hydroxyéthyl) amino-5 phénol, et leurs sels, les métaphénylènediamines telles que : le (diamino-2,4) phénoxyéthanol, le [N-(β-hydroxyéthyl) amino-2 amino-4] phénoxyéthanol, le (diamino-2,4) phényl β,γ-di-hydroxypropyléther et leurs sels, les métaacylaminophénols, les métauréidophénols, les métacarbalcoxyaminophénols tels que : le méthyl-2 acétylamino-5 phénol, le méthyl-2 uréido-5 phénol, le méthyl-2 carbéthoxy-amino-5 phénol.

On peut enfin mentionner comme autres coupleurs utilisables dans les compositions de l'invention : l'α-naphtol, les coupleurs possédant un groupement méthylène actif tels que les composés dicétoniques et les pyrazolones et les coupleurs hétérocycliques tels que la diamino-2,4-pyridine, l'hydroxy-6 benzomorpholine, l'amino-6 benzomorpholine ainsi que leurs sels.

Ces compositions contiennent, en plus des précurseurs de colorants par oxydation, des agents réducteurs tels que plus particulièrement le bisulfite de sodium, le sulfite de sodium, l'acide thioglycolique, l'acide thiolactique, l'acide mercapto succinique, l'acide ascorbique et leurs sels et l'hydroquinone. Ces agents réducteurs sont présents dans des proportions comprises entre 0,05 et 3 % en poids par rapport au poids total de la composition.

Les précurseurs de colorants par oxydation peuvent être utilisés, dans les compositions de l'invention, à des concentrations comprises entre 0,001 et 5 % en poids et de préférence entre 0,03 et 2 % en poids sur la base du poids total de la composition. Les coupleurs peuvent également être présents dans des proportions comprises entre 0,001 et 5 % en poids, de préférence entre 0,015 et 2 % en poids. Le pH de ces compositions de teinture par oxydation est de préférence compris entre 7 et 11,5 et est ajusté à l'aide d'agents alcalinisants définis ci-dessus.

La demanderesse a constaté que les composés conformes à l'invention étaient particulièrement stables dans de telles compositions.

Le procédé de teinture des fibres kératiniques, en particulier des cheveux humains, mettant en œuvre la révélation par un oxydant, consiste à appliquer sur les cheveux la composition tinctoriale comprenant à la fois un colorant selon l'invention et les précurseurs de colorants. Le développement de la coloration peut alors s'effectuer lentement en présence de l'oxygène de l'air, mais on utilise de préférence un système révélateur chimique qui est le plus souvent choisi parmi l'eau oxygénée, le peroxyde d'urée et les persels. On utilise en particulier une solution d'eau oxygénée à 20 volumes.

Une fois que l'on a appliqué sur les fibres kératiniques la composition avec l'agent oxydant, on laisse poser pendant 10 à 50 minutes, de préférence 15 à 30 minutes, après quoi on rince les fibres kératiniques, on les lave éventuellement au shampooing, on les rince à nouveau et on sèche.

Les exemples qui suivent sont destinés à illustrer l'invention sans présenter un caractère limitatif.

(Voir exemple de préparation 1 page 7)

Exemple de préparation 1

Préparation de la diméthyl-2,6 nitro-3 paraphénylènediamine

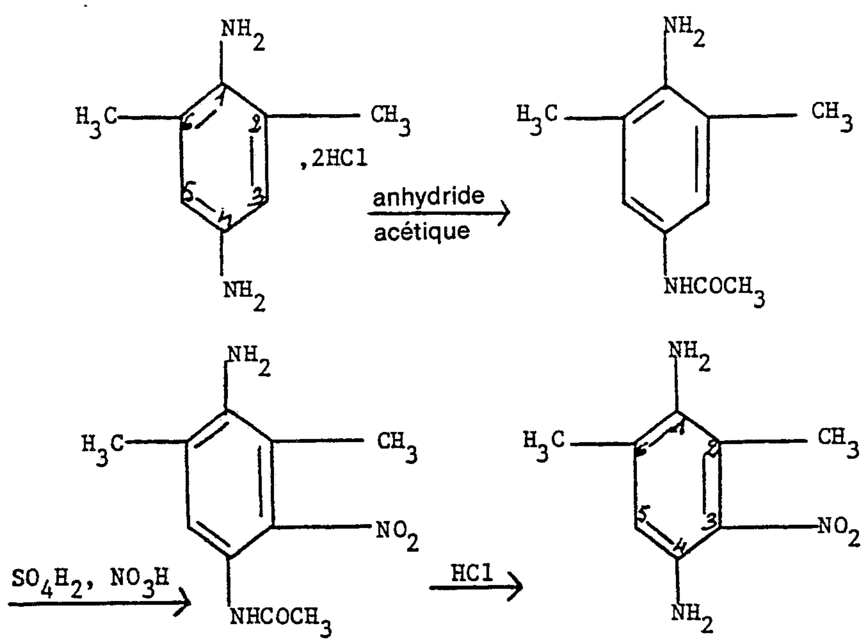


Première étape

Préparation de la diméthyl-2,6 acétylamino-4 aniline

On dissout 1,6 mole (334,6 g) de dichlorhydrate de diméthyl-2,6 paraphénylènediamine dans 1 600 ml d'eau. A cette solution on ajoute 3,36 moles (423,3 g) de sulfite de sodium en solution dans 1 600 ml d'eau puis, peu à peu, sous agitation, en maintenant la température au voisinage de 25 °C, 1,68 mole (174,8 g) d'anhydride acétique. L'addition terminée on abandonne le milieu réactionnel 1 heure à température ambiante. Après refroidissement, on essore le dérivé monoacétylé qui a précipité. Il fond à 148 °C.

Deuxième étape

Préparation de la diméthyl-2,6 nitro-3 acétylamino-4 aniline

On ajoute peu à peu, sous bonne agitation, 0,226 mole (40,3 g) du dérivé monoacétylé précédemment obtenu à 230 ml d'acide sulfurique à 96 % tout en maintenant la température entre 18 et 20 °C. La dissolution est complète au bout de 30 minutes. On refroidit alors cette solution à — 3 °C puis on lui ajoute goutte à goutte en 45 minutes, sous bonne agitation et tout en maintenant la température entre — 2 et 0 °C le mélange sulfonitrique préparé à partir de 10,5 ml d'acide nitrique (d = 1,5) et de 40 ml d'acide sulfurique à 96 %. L'addition terminée, on maintient le milieu réactionnel 1 heure à 0 °C puis on le verse sur 2 kg de glace broyée. Après neutralisation à l'aide d'ammoniaque, on essore le produit attendu qui a précipité, on le lave à l'eau et on le recristallise dans l'éthanol. Il fond à 197 °C.

| Analyse | Calculé pour $C_{10}H_{13}N_3O_3$ | Trouvé |
|---|---|---|
| C% | 53,80 | 53,83 |
| H% | 5,87 | 5,92 |
| N% | 18,83 | 18,93 |
| O% | 21,50 | 21,35 |

Troisième étape

Préparation de la diméthyl-2,6 nitro-3 paraphénylènediamine

On introduit 0,04 mole (8,9 g) de diméthyl-2,6, nitro-3, acétylamino-4 aniline dans 25 ml d'acide

chlorhydrique à 36 % additionnés de 10 ml d'acide acétique. Après 1 heure 1/2 de chauffage au bain-marie bouillant, on essore le produit attendu sous forme de chlorhydrate. Ce chlorhydrate est mis en solution dans 40 ml d'eau. Par addition d'ammoniaque on précipite la diméthyl-2,6 nitro-3, paraphénylènediamine. Le produit est essoré, lavé à l'eau, séché et recristallisé dans le benzène. Il fond à 124 °C.

| Analyse | Calculé pour | Trouvé |
|---|---|---|
| | $C_8H_{11}N_3O_2$ | |
| C% | 53,03 | 53,23 |
| H% | 6,12 | 6,14 |
| N% | 23,19 | 23,22 |
| O% | 17,66 | 17,58 |

Exemple de préparation 2

Préparation de la diméthyl-5,6 nitro-3 paraphénylènediamine

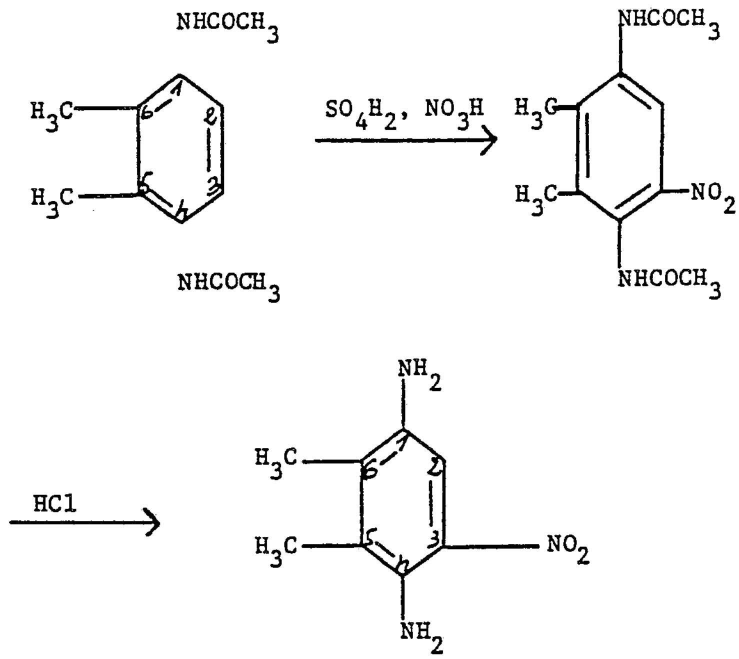


Première étape

Préparation de la diméthyl-5,6 nitro-3 N,N'-diacétylparaphénylènediamine

On ajoute peu à peu, sous bonne agitation, 0,085 mole (18,3 g) de diméthyl-5,6 N,N'-diacétylparaphénylènediamine à 85 ml d'acide sulfurique à 96 %, tout en maintenant la température entre 18 et 20 °C. La dissolution est totale au bout de 30 minutes. On refroidit cette solution à — 5 °C et on lui ajoute alors goutte à goutte en 20 minutes, tout en maintenant la température au voisinage de — 2 °C, le mélange sulfonitrique préparé selon le procédé habituel, à partir de 15 ml d'acide sulfurique à 96 % et de 4 ml d'acide nitrique d = 1,5. L'addition terminée, on maintient l'agitation 1 heure au voisinage de 0 °C puis on verse le milieu réactionnel sur 0,8 kg de glace pilée. On essore le produit attendu qui a précipité, on le lave à l'eau et le sèche sous vide à 50 °C. Après recristallisation dans l'éthanol, il fond au-dessus de 260 °C.

| Analyse | Calculé pour | Trouvé |
|---|---|---|
| | $C_{12}H_{15}N_3O_4$ | |
| C% | 54,33 | 54,36 |
| H% | 5,70 | 5,70 |
| N% | 15,84 | 15,93 |
| O% | 24,13 | 24,30 |

Deuxième étape

Préparation de la diméthyl-5,6 nitro-3 paraphénylènediamine

On chauffe 1 heure 1/2 au bain-marie bouillant 0,075 mole (19,9 g) du dérivé diacétylé préparé selon le procédé de la première étape, dans 75 ml d'acide chlorhydrique à 36 %.

Après refroidissement du milieu, on essore le produit attendu sous forme de chlorhydrate. On dissout ce chlorhydrate dans 150 ml d'eau et on alcalinise à l'aide d'ammoniaque pour précipiter la diméthyl-5,6 nitro-3 paraphénylènediamine. Le produit est essoré, lavé à l'eau, recristallisé à l'aide d'acétate d'éthyle ; il fond à 159 °C.

| Analyse | Calculé pour $C_8H_{11}N_3O_2$ | Trouvé |
|---|---|---|
| C% | 53,03 | 52,96 |
| H% | 6,12 | 6,08 |
| N% | 23,19 | 23,14 |
| O% | 17,66 | 17,71 |

Exemple de préparation 3

Préparation de l'amino-4 diméthyl-2,6 nitro-3 N-méthylaniline

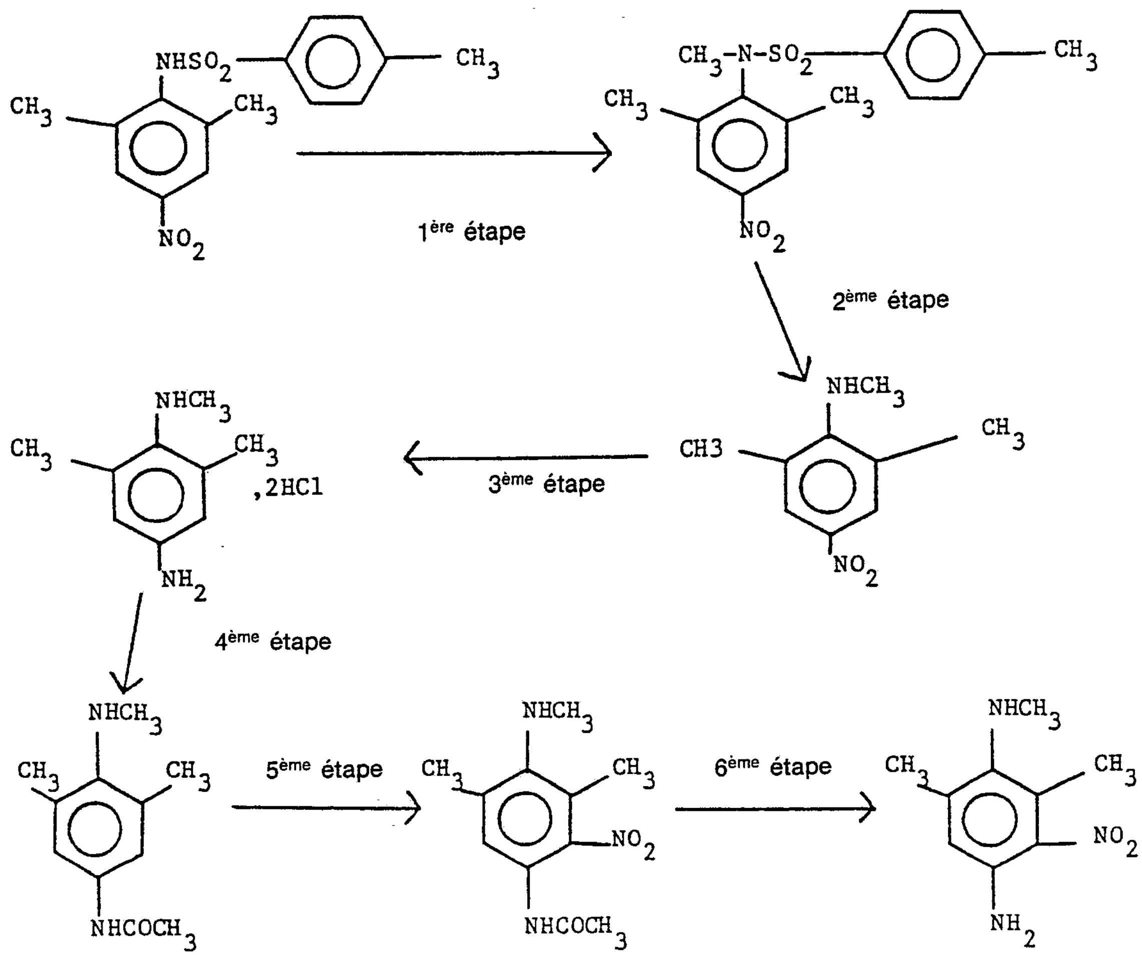


1re étape

Préparation du N,N-(méthyl, p-toluènesulfonyl) amino-1 diméthyl-2,6 nitro-4 benzène

On dissout 0,053 mole (16,9 g) de p-toluènesulfonylamino-1 diméthyl-2,6 nitro-4 benzène préparé selon BM WEPSTER, Recueil Tr. Chimiques, Pays-Bas, 73.809 [1954] dans 120 ml de soude N/2. On ajoute

peu à peu, à 20 °C, 0,06 mole (6 g) de diméthylsulfate. On agite 1 heure supplémentaire.

Après essorage et réempâtage dans l'eau du précipité, on obtient un produit qui recristallise de l'éthanol et fond à 128 °C.

L'analyse du produit obtenu donne les résultats suivants :

| Analyse | % calculé pour $C_{16}H_{18}N_2O_4S$ | % trouvé |
|---|---|---|
| C | 57,48 | 57,41 |
| H | 5,43 | 5,35 |
| N | 8,38 | 8,29 |
| O | 19,15 | 19,31 |
| S | 9,57 | 9,58 |

2e étape

Préparation de la diméthyl-2,6 nitro-4 N-méthylaniline

A une solution de 25 ml d'acide sulfurique à 96 % et 2,5 ml d'eau portée à 60 °C, on ajoute peu à peu 0,031 mole (10,4 g) de N,N-(méthyl p-toluènesulfonyl) amino-1 diméthyl-2,6 nitro-4 benzène. Après 30 minutes de chauffage supplémentaire, le milieu réactionnel est versé sur 90 g de glace. On essore le sulfate du produit attendu.

On met en suspension le sulfate dans l'eau puis on ajoute de l'ammoniaque à 20 % ; le produit attendu cristallise. Après recristallisation de l'alcool à 96 °, il fond à 99 °C.

L'analyse du produit obtenu donne les résultats suivants :

| Analyse | % calculé pour $C_9H_{12}N_2O_2$ | % trouvé |
|---|---|---|
| C | 59,98 | 60,06 |
| H | 6,71 | 6,75 |
| N | 15,55 | 15,50 |
| O | 17,76 | 17,77 |

3e étape

Préparation du dichlorhydrate d'amino-4 diméthyl-2,6 N-méthylaniline

On chauffe au reflux une suspension de 15,5 g de zinc en poudre et de 0,6 g de chlorure d'ammonium. On ajoute, après avoir enlevé le chauffage, 0,02 mole (3,6 g) de diméthyl-2,6 nitro-4 N-méthylaniline de façon à maintenir le reflux. A la fin de l'addition, le milieu réactionnel est filtré bouillant sur une solution de 0,05 mole d'acide chlorhydrique dans 14 ml d'éthanol absolu ; le dichlorhydrate cristallise du filtrat. Après recristallisation d'un mélange alcool-eau, on obtient un produit blanc :

— Masse moléculaire calculée pour $C_9H_{16}N_2Cl_2$ : 223

— Masse moléculaire trouvée par dosage potentiométrique dans l'eau par la soude : 220.

L'analyse du produit obtenu donne les résultats suivants :

| Analyse | % calculé pour $C_9H_{16}N_2Cl_2$ | % trouvé |
|---|---|---|
| C | 48,44 | 48,51 |
| H | 7,23 | 7,28 |
| N | 12,55 | 12,47 |
| Cl | 31,78 | 31,77 |

4e étape

Préparation du N-méthylamino-4 diméthyl-3,5 acétanilide

A une solution de 0,17 mole (38 g) de dichlorhydrate d'amino-4 diméthyl-2,6 N-méthylaniline dans 170 ml d'eau, on ajoute en une fois 0,37 mole (46,6 g) de sulfite de sodium dissous dans 170 ml d'eau puis goutte à goutte pendant 30 minutes, 0,183 mole (19 g) d'anhydride acétique, la température étant maintenue entre 18 °C et 23 °C. Après 2 heures d'agitation, on filtre et on neutralise le filtrat par de l'ammoniaque à 20 %. Après essorage, lavage à l'eau, séchage sous vide, on obtient 0,12 mole (23,3 g) du produit attendu ; il fond à 82 °C.

— Masse moléculaire calculée pour $C_{11}H_{16}N_2O$ : 192,3.

— Masse moléculaire trouvée par dosage potentiométrique dans l'acide acétique par l'acide perchlorique : 195.

5e étape

Préparation du N-méthylamino-4 diméthyl-3,5 nitro-2 acétanilide

On ajoute peu à peu une solution de 5,6 ml d'acide nitrique (d = 1,50) dans 20 ml d'acide sulfurique concentré à 0,12 mole (23 g) de N-méthyl-amino-4 diméthyl-3,5 acétanilide dans 120 ml d'acide sulfurique concentré en maintenant la température à 0 °C. Après 30 minutes d'agitation supplémentaire, le milieu réactionnel est versé sur 1 kg de glace. Après neutralisation par de l'ammoniaque à 20 %, le produit attendu précipite. Essoré et recristallisé avec de l'alcool à 96 °, il fond à 133 °C.

— Masse moléculaire calculée pour $C_{11}H_{15}N_3O_3$ : 237

— Masse moléculaire trouvée par dosage potentiométrique par l'acide perchlorique dans l'acide acétique : 240.

L'analyse du produit obtenu donne les résultats suivants :

| Analyse | % calculé pour $C_{11}H_{15}N_3O_3$ | % trouvé |
|---|---|---|
| C | 55,68 | 55,55 |
| H | 6,37 | 6,40 |
| N | 17,71 | 17,80 |
| O | 20,23 | 20,00 |

6e étape

Préparation de l'amino-4 diméthyl-2,6 nitro-3 N-méthylaniline

On ajoute 0,11 mole (26,0 g) de N-méthylamino-4 diméthyl-3,5 nitro-2 acétanilide à 65 ml d'acide chlorhydrique concentré. Le mélange est chauffé 1 heure à 95 °C. Après refroidissement, on essore le dichlorhydrate du produit attendu.

Le dichlorhydrate ainsi obtenu est mis en suspension dans 400 ml d'eau. On neutralise le milieu par de l'ammoniaque à 20 %, le produit attendu cristallise. Après essorage et séchage sous vide en présence de pentaoxyde de phosphore, il est recristallisé d'un mélange cyclohexane-benzène ; il fond à 57 °C.

— Masse moléculaire calculée pour $C_9H_{13}N_3O_2$ : 195

— Masse moléculaire trouvée par dosage potentiométrique dans l'acide acétique par l'acide perchlorique : 195.

L'analyse du produit obtenu donne les résultats suivants :

| Analyse | % calculé pour $C_9H_{13}N_3O_2$ | % trouvé |
|---|---|---|
| C | 55,37 | 55,43 |
| H | 6,71 | 6,69 |
| N | 21,53 | 21,54 |
| O | 16,39 | 16,41 |

Exemple de préparation 4

Préparation de l'amino-4 diméthyl-5,6 nitro-3 N-β-hydroxyéthylaniline

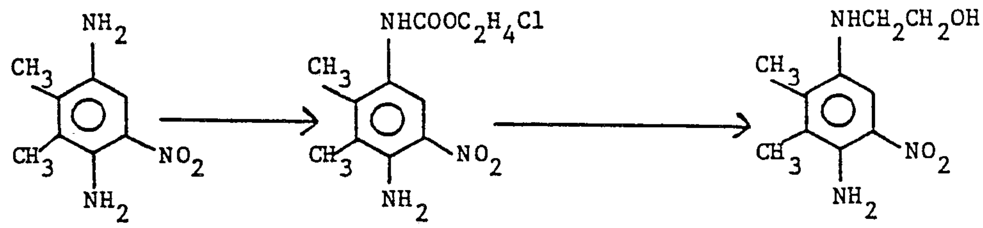

1ère étape

Préparation de l'[amino-4 diméthyl-5,6 nitro-3 phényl] carbamate de β-chloréthyle

On dissout 0,05 mole (9,1 g) de diméthyl-5,6 nitro-3 paraphénylène diamine dans 40 ml de dioxane. On ajoute 0,055 mole (5,5 g) de carbonate de calcium. On élève la température à 90 °C puis on introduit peu à peu sous agitation 0,053 mole (7,6 g) de chloroformiate de β-chloréthyle. Le chauffage est poursuivi 1 heure après la fin de l'addition. On verse le milieu réactionnel sur 200 g d'eau glacée. Le produit attendu précipite. Après essorage, réempâtage dans l'eau et recristallisation de l'éthanol à 96°, il fond à 151 °C.

L'analyse du produit obtenu donne les résultats suivants :

| Analyse | % calculé pour $C_{11}H_{14}N_3O_4Cl$ | % trouvé |
|---|---|---|
| C | 45,92 | 46,10 |
| H | 4,90 | 4,89 |
| N | 14,61 | 14,50 |
| O | 22,24 | 22,25 |

2e étape

Préparation de l'amino-4 diméthyl-5,6 nitro-3 N-β-hydroxyéthylaniline

On met en suspension dans 36 ml d'alcool à 96° 0,041 7 mole (12 g) d'[amino-4 diméthyl-5,6 nitro-3 phényl] carbamate de β-chloréthyle. On ajoute peu à peu sous agitation une solution de 6,2 g de soude en pastilles à 97 % dans 15 ml d'eau ; le mélange réactionnel est porté au reflux de l'alcool. Après 2 heures de chauffage, le milieu réactionnel est versé sur 200 g d'eau glacée et acidifié par de l'acide acétique.

Après essorage, réempâtage dans l'eau et recristallisation de l'acétate d'éthyle, le produit fond à 166 °C.

— Masse moléculaire calculée pour $C_{10}H_{15}N_3O_3$ : 225,25

— Masse moléculaire trouvée par dosage potentiométrique dans l'acide acétique par l'acide perchlorique : 223.

L'analyse du produit obtenu donne les résultats suivants :

| Analyse | % calculé pour $C_{10}H_{15}N_3O_3$ | % trouvé |
|---|---|---|
| C | 53,32 | 53,27 |
| H | 6,71 | 6,77 |
| N | 18,66 | 18,76 |
| O | 21,31 | 21,25 |

Exemple de préparation 5

Préparation de l'amino-4 diméthyl-5,6 nitro-3 N-β,γ-dihydroxypropylaniline

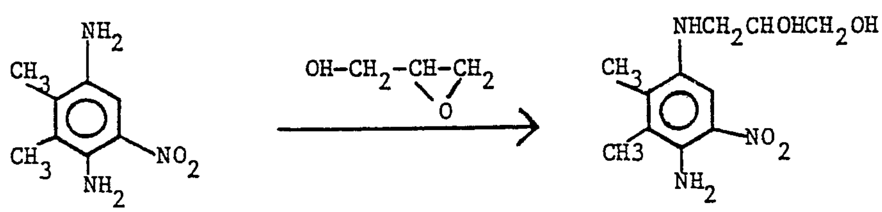

On chauffe au bain-marie bouillant sous agitation 0,05 mole (9 g) de diméthyl-5,6 nitro-3 paraphénylènediamine et 0,1 mole (7,4 g) de glycidol dans 18 ml d'éthanol.

Après 2 heures de chauffage, le milieu réactionnel est refroidi, le précipité est essoré, lavé à l'eau, à l'alcool puis séché. Après dissolution dans le diméthylsulfoxyde, puis reprécipitation par ajout d'eau et séchage, il fond à 181 °C.

L'analyse du produit obtenu donne les résultats suivants :

| Analyse | % calculé pour $C_{11}H_{17}N_3O_4$ | % trouvé |
|---|---|---|
| C | 51,76 | 51,77 |
| H | 6,67 | 6,70 |
| N | 16,47 | 16,34 |
| O | 25,10 | 24,91 |

Exemple de préparation 6

Préparation de l'amino-4 diméthyl-5,6 nitro-3 N-β-aminoéthylaniline

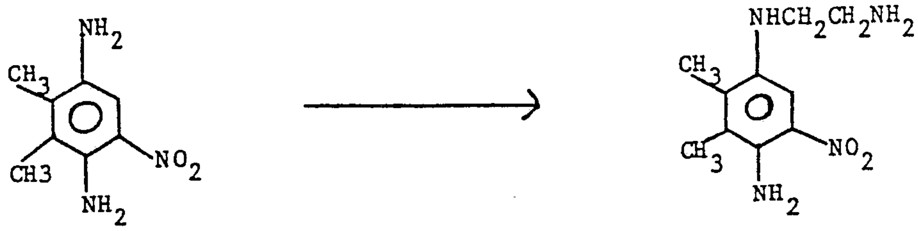

On chauffe préalablement à 80 °C sous agitation une suspension de 0,5 mole (90 g) de diméthyl-5,6 nitro-3 paraphénylènediamine et de 0,75 mole (75 g) de carbonate de chaux dans 350 ml d'eau et 150 ml d'alcool à 96°. On ajoute peu à peu, sous agitation, 1,2 mole (262 g) de bromhydrate de bromoéthylamine. Après 1 heure 30 de chauffage au reflux, on refroidit le milieu réactionnel et on coule 250 ml d'acide chlorhydrique concentré, le précipité ainsi obtenu est essoré, lavé à l'eau puis à l'acétone. Après dissolution dans 1 litre d'eau glacée par addition d'ammoniaque, on obtient le sel du produit attendu qui, après alcalinisation par 250 ml de soude 10N, conduit à l'amino-4 diméthyl-5,6 nitro-3 N-β-aminoéthylaniline. Après lavage à l'eau, séchage et recristallisation dans l'alcool éthylique, le produit fond à 152 °C.

| Analyse | % calculé pour $C_{10}H_{16}N_4O_2$ | % trouvé |
|---|---|---|
| C | 53,57 | 53,62 |
| H | 7,14 | 7,18 |
| N | 25,00 | 24,91 |
| O | 14,29 | 14,12 |

Exemple de préparation 7

Préparation de l'amino-4 diméthyl-5,6 nitro-3 N-(β-diéthylaminoéthyl) aniline

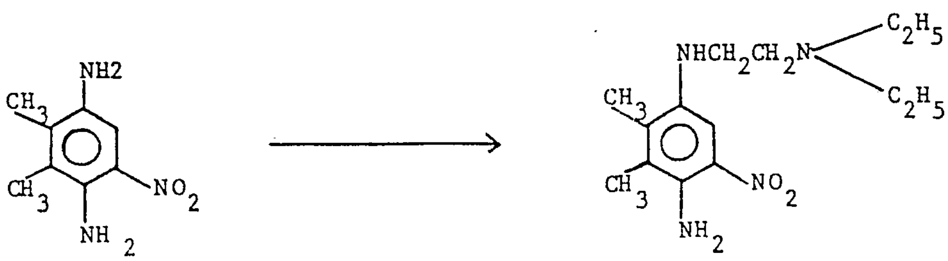

On chauffe à 80 °C, sous agitation, un mélange constitué de 0,05 mole (9 g) de diméthyl-5,6 nitro-3

paraphénylènediamine, 0,075 mole (7,5 g) de carbonate de chaux et de 0,1 mole (17,2 g) de chlorhydrate de bromo-1 N,N-diéthylamino-2 éthane dans 36 ml d'eau et 18 ml d'éthanol à 96°. Après 45 minutes de chauffage, on filtre le milieu réactionnel à chaud, et on ajoute 25 ml d'acide chlorhydrique au filtrat. Le chlorhydrate du produit attendu précipite. On l'essore et on le lave à l'acétone.

Le chlorhydrate est dissous dans l'eau, et par addition de soude 10N en présence de glace, on obtient une huile qui précipite. Après essorage, lavage à l'eau et séchage en présence de pentaoxyde de phosphore sous vide, le produit obtenu est recristallisé de l'éthanol à 96 °C. Il fond à 98 °C.

L'analyse du produit obtenu donne les résultats suivants :

| Analyse | % calculé pour $C_{14}H_{24}N_4O_2$ | % trouvé |
|---|---|---|
| C | 60,00 | 59,96 |
| H | 8,57 | 8,46 |
| N | 20,00 | 20,02 |
| O | 11,43 | 11,67 |

Exemple 1

Teinture directe

On prépare la composition tinctoriale suivante :

| | |
|---|---|
| — amino-4 diméthyl-2,6 nitro-3N-méthylaniline | 0,3 g |
| — amino-4 méthyl-2 nitro-3N-β-hydroxyéthylaniline | 0,12 g |
| — dichlorhydrate de nitro-3N'-β-aminoéthylamino-4 N,N-(β-hydroxyéthyl) aniline | 0,18 g |
| — amino-2 nitro-3 phénol | 0,12 g |
| — propylèneglycol | 10 g |
| — hydroxyéthylcellulose vendue sous la dénomination CELLOSIZE WPO3 par UNION CARBIDE | 2 g |
| — chlorure de cétyldiméthylhydroxyéthylammonium vendu sous le nom de CHIMEXON par la Société CHIMEX | |
| — Ammoniaque à 5 % | 4 ml |
| — Eau qsp | 100 g |
| — pH 9,2 | |

Ce mélange, appliqué 20 minutes à 28 °C sur cheveux naturellement blancs à 90 % permanentés, leur confère, après shampooing et rinçage, une coloration acajou.

Exemple 2

Teinture directe

On prépare la composition tinctoriale suivante :

| | |
|---|---|
| — amino-4 diméthyl-2,6 nitro-3N-méthylaniline | 0,055 g |
| — alcool à 96° | 10 g |
| — ALFOL C16/18E (alcool cétylstéarylique vendu par la Société CONDEA) | 8 g |
| — Cire de lanette E (sulfate cétylstéarylique de Na vendu par HENKEL) | 0,5 g |
| — CEMULSOL B (huile de ricin éthoxylée vendue par RHONE-POULENC) | 1 g |
| — diéthanolamide oléïque | 1,5 g |
| — ammoniaque 5 % | 1 g |
| — Eau qsp | 100 g |
| — pH 9,8 | |

Ce mélange, appliqué 30 minutes à 28 °C sur cheveux décolorés au blanc leur confère, après shampooing et rinçage, une coloration jaune d'or clair.

Exemple 3

Teinture directe

On prépare la composition tinctoriale suivante :

| | |
|---|---|
| — amino-4 diméthyl-5,6 nitro-3N-β-hydroxyéthylaniline | 0,385 g |
| — N-β-hydroxyéthylamino-2 nitro-5 phénol | 0,085 g |
| — nitro-3 N'-β-hydroxyéthylamino-4 N-méthyl, N-β-hydroxyéthylaniline | 0,15 g |
| — butoxy-2 éthanol | 10 g |
| — ALFOL C16/18E (alcool cétylstéarylique vendu par la Société CONDEA) | 8 g |
| — Cire de lanette E (sulfate cétylstéarylique de Na vendu par HENKEL) | 0,5 g |
| — CEMULSOL B (huile de ricin éthoxylée vendue par RHONE-POULENC) | 1 g |
| — diéthanolamide oléïque | 1,5 g |
| — triéthanolamine en solution à 1 % | 2 ml |
| — Eau qsp | 100 g |
| — pH 7,6 | |

Ce mélange, appliqué 25 minutes à 28 °C sur cheveux décolorés au blanc, leur confère, après shampooing et rinçage, une coloration cuivre clair.

Exemple 4

Teinture directe

On prépare la composition tinctoriale suivante :

| | |
|---|---|
| — amino-4 diméthyl-5,6 nitro-3N-β-hydroxyéthylaniline | 2 g |
| — butoxy-2 éthanol | 25 g |
| — CARBOPOL 934 de GOODRICH CHEMICAL Cie (polymère de l'acide acrylique PM : 2 à 3 millions) | 2 g |
| — triéthanolamine pure | 4,5 g |
| — Eau qsp | 100 g |
| — pH 7,6 | |

Ce mélange, appliqué 15 minutes à 30 °C sur cheveux naturellement blancs à 90 %, leur confère, après shampooing et rinçage, une coloration beige rosé.

Exemple 5

Teinture directe

On prépare le mélange tinctorial suivant :

| | |
|---|---|
| — amino-4 diméthyl-5,6 nitro-3N-β,γ-dihydroxy-propylaniline | 0,25 g |
| — butoxy-2 éthanol | 6 g |
| — LAURAMIDE — Société WITCO (monoéthanolamide d'acide laurique) | 1,5 g |
| — acide laurique | 1 g |
| — CELLOSIZE W.P.03 — Société UNION CARBIDE (hydroxyéthylcellulose) | 5 g |
| — monoéthanolamine | 2 g |
| — Eau qsp | 100 g |
| — pH 9,3 | |

Ce mélange, appliqué 20 minutes à 28 °C sur cheveux décolorés au blanc, leur confère, après shampooing et rinçage, une coloration 2,5 R 8/6 selon la notation de Munsell.

Exemple 6

Teinture directe

On prépare le mélange tinctorial suivant :

| | |
|---|---|
| — amino-4 diméthyl-5,6 nitro-3N-β,γ-dihydroxypropyl aniline | 0,2 g |
| — chlorhydrate de N'-(β-hydroxyéthyl) amino-4 nitro-3 N,N-bis-(β-hydroxyéthyl) aniline | 0,05 g |
| — amino-4 nitro-3 phénol | 0,1 g |
| — butoxy-2 éthanol | 10 g |
| — ALFOL C 16/18 E — Société CONDEA (alcool cétylstéarylique) | 8 g |
| — Cire de Lanette E — Société HENKEL (sulfate cétylstéarylique de sodium) | 0,5 g |
| — CEMULSOL B — Société RHONE-POULENC (huile de ricin éthoxylée) | 1 g |
| — diéthanolamide oléïque | 1,5 g |
| — Eau qsp | 100 g |
| — pH 7,2 | |

Ce mélange, appliqué 20 minutes à 30 °C sur cheveux naturellement blancs à 90 %, leur confère, après shampooing et rinçage, une coloration miel doré.

Exemple 7

Teinture directe

On prépare le mélange tinctorial suivant :

| | |
|---|---|
| — amino-4 diméthyl-5,6 nitro-3 N-β-aminoéthyaniline | 0,5 g |
| — alcool éthylique à 96° | 10 g |
| — COMPERLAN KD — Société HENKEL (diéthanolamide d'acide gras de coprah) | 2,2 g |
| — acide laurique | 0,8 g |
| — monoéther éthylique de l'éthylèneglycol | 2 g |
| — monoéthanolamine | 6 g |
| — Eau qsp | 100 g |
| — pH 10 | |

Ce mélange, appliqué 20 minutes à 28 °C sur cheveux, leur confère, après shampooing et rinçage, une coloration :

— sur cheveux décolorés au blanc 10 RP 5/8

— sur cheveux naturellement blancs à 90 % 10 RP 3/10 selon la notation de Munsell.

Exemple 8

Teinture directe

On prépare le mélange tinctorial suivant :

| | |
|---|---|
| — amino-4 diméthyl-5,6 nitro-3 N-β-aminoéthylaniline | 0,01 g |
| — [N-(β-hydroxyéthyl)amino-4 nitro-3] phényl-β-hydroxyéthyléther | 0,25 g |
| — N'-(β-hydroxyéthyl) amino-4 nitro-3 N,N-bis-(β-hydroxyéthyl) aniline | 0,20 g |
| — butoxy-2 éthanol | 10 g |
| — CEMULSOL NP 4 — Société RHONE-POULENC (nonylphénol à 4 moles d'oxyde d'éthylène) | 12 g |
| — CEMULSOL NP 9 — Société RHONE POULENC (nonylphénol à 9 moles d'oxyde d'éthylène) | 15 g |
| — alcool oléïque polyglycérolé à 2 moles de glycérol | 1,5 g |
| — alcool oléïque polyglycérolé à 4 moles de glycérol | 1,5 g |
| — acide lactique | 0,5 g |
| — Eau qsp | 100 g |
| — pH 5 | |

Ce mélange, appliqué 30 minutes à 30 °C sur cheveux naturellement blancs à 90 %, leur confère, après shampooing et rinçage, une coloration beige rosé.

Exemple 9

Teinture d'oxydation

On prépare le mélange tinctorial suivant :

| | |
|---|---|
| — amino-4 diméthyl-5,6 nitro-3 N-β,γ-dihydroxypropyl aniline | 0,1 g |
| — paraphénylènediamine | 0,05 g |
| — paraaminophénol | 0,27 g |
| — sulfate de N-méthylamino-4 phénol | 0,12 g |
| — dichlorhydrate de diamino-2,4 phénoxyéthanol | 0,017 g |
| — résorcine | 0,06 g |
| — métaaminophénol | 0,055 g |
| — isopropyl-2 nitro 6 aniline | 0,04 g |
| — CEMULSOL NP 4 (nonylphénol à 4 moles d'oxyde d'éthylène vendu par RHONE POULENC) | 21 g |
| — CEMULSOL NP 9 (nonylphénol à 9 moles d'oxyde d'éthylène vendu par RHONE POULENC) | 24 g |
| — Acide oléïque | 4 g |
| — butoxy-2 éthanol | 3 g |

| | |
|---|---|
| — éthanol à 96° | 10 g |
| — MASQUOL DTPA (sel de sodium de l'acide diéthylènetriamine pentacétique) | 2,5 g |
| — acide thioglycolique | 0,6 g |
| — ammoniaque à 22° Bé | 10 g |
| — Eau qsp | 100 g |
| — pH 10,1 | |

Au moment de l'emploi, on ajoute 100 g d'eau oxygénée à 20 volumes.

Ce mélange, appliqué 20 minutes à 30 °C sur des cheveux permanentés, leur confère, après shampooing et rinçage, une coloration châtain clair doré.

Exemple 10

Teinture d'oxydation

On prépare la composition tinctoriale suivante :

| | |
|---|---|
| — amino-4 diméthyl-5,6 nitro-3 N-β-aminoéthylaniline | 0,15 g |
| — (N-méthylamino-3 nitro-4) phényl, β-hydroxyéthyléther | 0,2 g |
| — paraphénylènediamine | 0,08 g |
| — paraaminophénol | 0,1 g |
| — résorcine | 0,05 g |
| — métaaminophénol | 0,15 g |
| — carboxyméthylcellulose | 2 g |
| — laurylsulfate d'ammonium | 5 g |
| — butoxy-2 éthanol | 8 g |
| — propylèneglycol | 8 g |
| — MASQUOL DTPA | 2 g |
| — acide thioglycolique | 0,4 g |
| — acétate d'ammonium | 1 g |
| — ammoniaque à 22° Bé | 10 g |
| — Eau qsq | 100 g |
| — pH 9,8 | |

Au moment de l'emploi, on ajoute 100 g d'eau oxygénée à 20 volumes.

Ce mélange, appliqué sur des cheveux décolorés 25 minutes à 30 °C, leur confère, après rinçage et shampooing, une coloration châtain doré.

Exemple 11

Teinture directe

On prépare le mélange tinctorial suivant :

| | |
|---|---|
| — amino-4 diméthyl-5,6 nitro-3 N-(β-diéthylaminoéthyl) aniline | 0,25 g |
| — alcool à 96° | 10 g |
| — triéthanolamine à 1 % en poids | 3 g |
| — CELLOSIZE W.P. 03 — Société UNION CARBIDE (hydroxyéthylcellulose) | 2 g |
| — Laurylsulfate d'ammonium | 5 g |
| — Eau qsp | 100 g |
| — pH 8,37 | |

Ce mélange, appliqué 20 minutes à 28 °C sur cheveux décolorés, leur confère, après shampooing et rinçage, une coloration 9 R 7/5 selon la notation de Munsell.

Exemple 12

Teinture d'oxydation

On prépare le mélange tinctorial suivant :

| | |
|---|---|
| — amino-4 diméthyl-5,6 nitro-3 N-(β-diéthylaminoéthyl) aniline | 0,2 g |
| — orthoaminophénol | 0,064 g |
| — paraphénylènediamine | 0,3 g |
| — paraaminophénol | 0,08 g |

| | |
|---|---|
| — résorcine | 0,15 g |
| — métaaminophénol | 0,08 g |
| — CEMULSOL NP 4 (nonylphénol oxyéthyléné à 4 moles d'oxyde d'éthylène vendu par RHONE-POULENC) | 12 g |
| — CEMULSOL NP 9 (nonylphénol oxyéthyléné à 9 moles d'oxyde d'éthylène vendu par RHONE-POULENC) | 15 g |
| — alcool oléïque polyglycérolé à 2 moles de glycérol | 1,8 g |
| — alcool oléïque polyglycérolé à 4 moles de glycérol | 1,5 g |
| — propylèneglycol | 6 g |
| — TRILON B (sel de sodium de l'acide éthylènediamine tétraacétique) | 0,12 g |
| — ammoniaque à 22° Bé | 11 g |
| — acide thioglycolique | 0,6 g |
| — Eau qsp | 100 g |
| — pH 10,5 | |

Au moment de l'emploi, on ajoute un poids égal d'eau oxygénée à 20 volumes.

Ce mélange, appliqué 25 minutes à 28 °C sur cheveux permanentés, leur confère, après shampooing et rinçage, une coloration châtain moyen.

## Exemple 13

Teinture directe

On prépare le mélange tinctorial suivant :

| | |
|---|---|
| — amino-4 diméthyl-5,6 nitro-3 N-(β-diéthylaminoéthyl) aniline | 0,05 g |
| — tétraamino-1,4 5,8 anthraquinone | 0,15 g |
| — méthyl-2 amino-4 nitro-5 phénol | 0,1 g |
| — butoxy-2 éthanol | 6 g |
| — LAURAMIDE — Société WITCO (monoéthanolamide d'acide laurique) | 1,5 g |
| — acide laurique | 1 g |
| — CELLOSIZE W.P. 03 — Société UNION CARBIDE (hydroxyéthylcellulose) | 5 g |
| — monoéthanolamine | 2 g |
| — Eau qsp | 100 g |
| — pH 9,5 | |

Ce mélange, appliqué 30 minutes à 28 °C sur cheveux naturellement blancs à 90 %, leur confère, après shampooing et rinçage, une coloration sable.

## Exemple 14

Teinture directe

On prépare la composition tinctoriale suivante :

| | |
|---|---|
| Diméthyl-5,6 nitro-3 paraphénylènediamine | 0,15 g |
| Butoxy-2 éthanol | 15 g |
| Diéthanolamides d'acides gras de coprah | 2,2 g |
| Acide laurique | 0,8 g |
| Monoéther éthylique de l'éthylèneglycol | 2 g |
| Monoéthanolamine | 1 g |
| Eau q.s.p. | 100 g |
| pH = 7,0. | |

Ce mélange, appliqué 25 minutes à 28 °C sur des cheveux décolorés au blanc, leur confère, après rinçage et shampooing, une coloration saumon rose.

## Exemple 15

Teinture directe

On prépare la composition tinctoriale suivante :

| | |
|---|---|
| Diméthyl-2,6 nitro-3 paraphénylènediamine | 0,1 g |
| Butoxy-2 éthanol | 10 g |

| | |
|---|---|
| Hydroxyéthylcellulose | 2 g |
| Laurylsulfate d'ammonium | 5 g |
| Solution de triéthanoamine à 1 % | 2 g |
| Eau q.s.p. | 100 g |
| pH = 8,4. | |

Ce mélange, appliqué 20 minutes à 28 °C sur des cheveux décolorés au blanc, leur confère, après rinçage et shampooing, une coloration champagne rosé.

## Exemple 16

Teinture directe

On prépare la composition tinctoriale suivante :

| | |
|---|---|
| Diméthyl-5,6 nitro-3 paraphénylènediamine | 0,35 g |
| Amino-2 nitro-3 phénol | 0,12 g |
| Dichlorhydrate de nitro-3 N'-β-aminoéthylamino-4 N,N-(β-hydroxyéthyl) aniline | 0,15 g |
| Butoxy-2 éthanol | 10 g |
| Alfol $C_{16}/_{18E}$ (Alcool cétylstéarylique vendu par la Société Condéa) | 8 g |
| Cire de lanette E (sulfate cétylstéarylique de sodium vendu par Henkel) | 0,5 g |
| Cemulsol B (huile de ricin éthoxylée vendue par Rhône Poulenc) | 1 g |
| Diéthanolamide oléique | 1,5 g |
| Ammoniaque à 22° Bé | 2 g |
| Eau q.s.p. | 100 g |
| pH = 10,0 | |

Ce mélange, appliqué sur cheveux décolorés 15 minutes à 25 °C, leur confère, après rinçage et shampooing, une coloration cuivre rouge.

## Exemple 17

Teinture directe

On prépare la composition tinctoriale suivante :

| | |
|---|---|
| Diméthyl-5,6 nitro-3 paraphénylènediamine | 0,16 g |
| Diméthyl-2,6 nitro-3 paraphénylènediamine | 0,3 g |
| Méthyl-2 amino-4 nitro-5 phénol | 0,2 g |
| Méthyl-2 nitro-5 N,N'(β-hydroxyéthyl) paraphénylènediamine | 0,08 g |
| Tétraamino-1,4,5,8 anthraquinone | 0,25 g |
| Butoxy-2 éthanol | 10 g |
| Alfol $C_{16}/_{18}E$ (alcool cétylstéarique vendu par la Société Condéa) | 8 g |
| Cire de lanette E (sulfate cétylstéarylique de sodium vendu par Henkel) | 0,5 g |
| Cémulsol B (huile de ricin éthoxylée vendue par Rhône Poulenc) | 1 g |
| Diéthanolamide oléique | 1,5 g |
| Solution de méthyl-2 amino-2 propanol-1 à 25 % | 1 g |
| Eau q.s.p. | 100 g |
| pH 9,8 | |

Ce mélange, appliqué 20 minutes à 28 °C sur cheveux blancs à 90 % permanentés, leur confère, après rinçage et shampooing, une coloration châtain moyen cuivré.

## Exemple 18

Teinture directe

On prépare la composition tinctoriale suivante :

| | |
|---|---|
| Diméthyl-5,6 nitro-3 paraphénylènediamine | 0,25 g |
| Méthyl-2, nitro-6 aniline | 0,1 g |
| Tétraamino-1,4,5,8 anthraquinone | 0,25 g |
| Alcool à 96° | 20 g |
| Hydroxyéthylcellulose | 2 g |
| Laurylsulfate d'ammonium | 5 g |

| | |
|---|---|
| Ammoniaque à 5 % | 0,5 g |
| Eau q.s.p. | 100 g |
| pH = 8. | |

Ce mélange est appliqué 15 minutes à 30 °C sur des cheveux à 95 % blancs permanentés ; après rinçage et shampooing, il leur confère une coloration cuivre.

Exemple 19

Teinture directe

On prépare la composition tinctoriale suivante :

| | |
|---|---|
| Diméthyl-2,6, nitro-3 paraphénylènediamine | 0,25 g |
| Nitro-3 N'-β-hydroxyéthylamino-4 N-méthylaniline | 0,15 g |
| N-β-hydroxyéthylamino-2 nitro-5 anisole | 0,08 g |
| Butoxy-2 éthanol | 10 g |
| Lauramide (monoéthanolamide d'acide laurique vendu par Witco) | 1,5 g |
| Acide laurique | 1 g |
| Hydroxyéthylcellulose | 5 g |
| Monoéthanolamine | 2 g |
| Eau q.s.p. | 100 g |
| pH = 9,5. | |

Ce mélange, appliqué 20 minutes à 28 °C sur des cheveux naturellement blancs à 90 %, leur confère, après rinçage et shampooing, une coloration châtain clair cuivré.

Exemple 20

Teinture d'oxydation

On prépare la composition tinctoriale suivante :

| | |
|---|---|
| Paraphénylènediamine | 0,16 g |
| Résorcine | 0,1 g |
| Métaaminophénol | 0,05 g |
| Dichlorhydrate de diamino-2,4 phénoxyéthanol | 0,03 g |
| Diméthyl-5,6 nitro-3 paraphénylènediamine | 0,35 g |
| Méthyl-2 nitro-5 N,N'-(β-hydroxyéthyl) paraphénylènediamine | 0,08 g |
| Alcool oléique oxyéthyléné à 2 moles d'oxyde d'éthylène | 4,5 g |
| Alcool oléique oxyéthyléné à 4 moles d'oxyde d'éthylène | 4,5 g |
| Ethomeen $TO_{12}$ (oléylstéarylamine oxyéthylénée à 12 moles d'oxyde d'éthylène vendue par Armak | 4,5 g |
| Diéthanolamides d'acides gras de coprah | 9 g |
| Propylèneglycol | 4 g |
| Butoxy-2 éthanol | 8 g |
| Ethanol à 96° | 6 g |
| Masquol DTPA (sel de sodium de l'acide diéthylène triamine pentacétique) | 2 g |
| Hydroquinone | 0,15 g |
| Solution de bisulfite de sodium à 35° Bé | 1,3 g |
| Ammoniaque à 22° Bé | 10 g |
| Eau q.s.p. | 100 g |
| pH = 10,5. | |

Au moment de l'emploi, on ajoute 100 g d'eau oxygénée à 20 volumes.

Ce mélange appliqué 20 minutes à 25 °C sur des cheveux naturellement blancs à 90 % leur confère, après rinçage et shampooing, une coloration marron rouge.

Exemple 21

Teinture d'oxydation

On prépare la composition tinctoriale suivante :

| | |
|---|---|
| Paraphénylènediamine | 0,2 g |

| | |
|---|---|
| Dichlorhydrate de diamino-2,4 phénoxyéthanol | 0,07 g |
| Résorcine | 0,2 g |
| Métaaminophénol | 0,08 g |
| Méthyl-2-N-β-hydroxyéthylamino-5-phénol | 0,07 g |
| Paraaminophénol | 0,08 g |
| Diméthyl-2,6 nitro-3 paraphénylènediamine | 0,22 g |
| Cemulsol $NP_4$ (nonylphénol à 4 moles d'oxyde d'éthylène vendu par Rhône Poulenc) | 21 g |
| Cemulsol $NP_9$ (nonylphénol à 9 moles d'oxyde d'éthylène vendu par Rhône Poulenc) | 24 g |
| Acide oléique | 4 g |
| Butoxy-2 éthanol | 3 g |
| Ethanol à 96° | 10 g |
| Masquol DTPA | 2,5 g |
| Bisulfite de sodium à 35° Bé | 1 g |
| Ammoniaque à 22° Bé | 10 g |
| Eau q.s.p. | 100 g |
| pH = 10,5. | |

Au moment de l'emploi, on ajoute 100 g d'eau oxygénée à 20 volumes.

Ce mélange, appliqué 20 minutes à 30 °C sur des cheveux naturellement blancs à 90 %, leur confère, après rinçage et shampooing, une coloration brune à reflets violine.

Exemple 22

Teinture d'oxydation

On prépare la composition tinctoriale suivante :

| | |
|---|---|
| Dichlorhydrate de paratoluylènediamine | 0,23 g |
| Résorcine | 0,12 g |
| Méthyl-2 N-méthylamino-5 phénol | 0,08 g |
| Paraaminophénol | 0,05 g |
| Diméthyl-2,6 nitro-3 paraphénylènediamine | 0,4 g |
| Alfol $C_{16}/_{18}E$ (alcool cétylstéarylique vendu par la Société Condea) | 8 g |
| Cire de lanette E (sulfate cétylstéarylique de sodium vendu par Henkel) | 0,5 g |
| Cemulsol B (huile de ricin éthoxylée vendue par Rhône Poulenc) | 1 g |
| Diéthanolamide oléique | 1,5 g |
| Masquol DTPA | 2,5 g |
| Acide mercaptosuccinique | 0,3 g |
| Ammoniaque à 22° Bé | 11 g |
| Eau q.s.p. | 100 g |
| pH = 10,5. | |

Au moment de l'emploi, on ajoute 100 g d'eau oxygénée à 20 volumes.

Ce mélange, appliqué 25 minutes à 25 °C sur des cheveux décolorés au blanc, leur confère, après rinçage et shampooing, une coloration acajou.

Exemple 23

Teinture d'oxydation

On prépare la composition tinctoriale suivante :

| | |
|---|---|
| Diméthyl-5,6 nitro-3 paraphénylènediamine | 0,045 g |
| Paraphénylènediamine | 0,03 g |
| Résorcine | 0,06 g |
| Paraaminophénol | 0,02 g |
| Hydroxyéthylcellulose | 2 g |
| Laurylsulfate d'ammonium | 5 g |
| Butoxy-2 éthanol | 15 g |
| Alcool à 96° | 5 g |
| Ammoniaque à 20 % | 10 g |
| Bisulfite de sodium à 35° Bé | 1 g |
| Hydroquinone | 0,15 g |
| Eau q.s.p. | 100 g |
| pH = 10,3. | |

Au moment de l'emploi, on ajoute 75 g d'eau oxygénée à 20 volumes.

Ce mélange, appliqué 25 minutes à 20 °C sur des cheveux décolorés, leur confère, après rinçage et shampooing, une coloration sable d'or.

Exemple 24

Teinture d'oxydation

On prépare la composition tinctoriale suivante :

| | |
|---|---|
| Diméthyl-5,6 nitro-3 paraphénylènediamine | 0,2 g |
| Dichlorhydrate de N,N-di-β-hydroxyéthyl paraphénylènediamine | 0,08 g |
| Paraaminophénol | 0,15 g |
| Méthyl-2 N-β-hydroxyéthylamino-5 phénol | 0,1 g |
| Résorcine | 0,16 g |
| N'-β-hydroxyéthylamino-4 nitro-3 N-méthyl N-β-hydroxyéthylaniline | 0,1 g |
| Hydroxyéthylcellulose vendue sous la dénomination Cellosize WP 03 par Union Carbide | 2 g |
| Laurylsulfate d'ammonium | 5 g |
| Butoxy-2 éthanol | 15 g |
| Alcool à 96° | 5 g |
| Masquol DTPA | 2 g |
| Hydroquinone | 0,15 g |
| Solution de bisulfite de sodium d = 1,32 | 1,5 g |
| Monoéthanolamine | 8 g |
| Eau q.s.p. | 100 g |
| pH = 10,7. | |

Au moment de l'emploi, on ajoute 80 g d'eau oxygénée à 20 volumes.

Ce mélange, appliqué 25 minutes à 25 °C sur des cheveux décolorés, leur confère, après rinçage et shampooing, une coloration corail.

Exemple 25

Teinture d'oxydation

On prépare la composition tinctoriale suivante :

| | |
|---|---|
| Diméthyl-2,6 nitro-3 paraphénylènediamine | 0,2 g |
| Dichlorhydrate de diméthyl-2,6 paraphénylènediamine | 0,1 g |
| Dichlorhydrate de diamino-2,4 phénoxyéthanol | 0,04 g |
| Résorcine | 0,11 g |
| Métaaminophénol | 0,06 g |
| Méthyl-2 N-β-hydroxyéthylamino-5 phénol | 0,05 g |
| Paraaminophénol | 0,05 g |
| Remc opal 334 (nonylphénol à 4 moles d'oxyde d'éthylène vendu par Gerland) | 21 g |
| Remc opal 349 (nonylphénol à 9 moles d'oxyde d'éthylène vendu par Gerland) | 24 g |
| Acide oléique | 4 g |
| Butoxy-2 éthanol | 3 g |
| Alcool à 96° | 10 g |
| Masquol DTPA | 2,5 g |
| Bisulfite de sodium à 35° Bé | 1 g |
| Hydroquinone | 0,12 g |
| Ammoniaque à 22° Bé | 10 g |
| Eau q.s.p. | 100 g |
| pH = 10,6. | |

Au moment de l'emploi, on ajoute 100 g d'eau oxygénée à 20 volumes. Ce mélange, appliqué 20 minutes à 28 °C sur des cheveux décolorés, leur confère, après rinçage et shampooing, une coloration gris étain à reflets roses.

Exemple 26

Teinture d'oxydation

On prépare la composition tinctoriale suivante :

| | |
|---|---|
| Diméthyl-5,6 nitro-3 paraphénylènediamine | 0,25 g |
| Dichlorhydrate de N-(β-méthoxyéthyl) paraphénylènediamine | 1 g |
| Paraaminophénol | 0,3 g |
| Résorcine | 0,25 g |
| Métaaminophénol | 0,15 g |
| Hydroquinone | 0,15 g |
| Nonylphénol oxyéthyléné à 9 moles d'oxyde d'éthylène | 3 g |
| Alcool oléique | 9 g |
| Diéthanolamide oléique | 9 g |
| Amide de suif hydrogéné à 50 moles d'oxyde d'éthylène | 2,5 g |
| Acide oléique | 18 g |
| Polymère cationique présentant le motif récurrent : | 3 g |

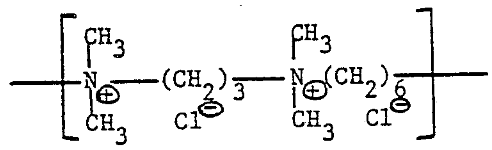

| | |
|---|---|
| Alcool éthylique | 9 g |
| Alcool benzylique | 11 g |
| Acide éthylène-diamino-tétracétique | 0,2 g |
| Ammoniaque à 22° Bé | 14 g |
| Monoéthanolamine | 6,5 g |
| Bisulfite de sodium à 35° Bé | 1,3 g |
| Eau q.s.p. | 100 g |

Cette composition liquide donne un gel crémeux par dilution poids pour poids avec de l'eau oxygénée à 20 volumes.

Si l'on applique 30 minutes ce gel sur une chevelure blond foncé et si l'on effectue finalement un shampooing, on obtient, après séchage, une coloration de cheveux blond clair rouge cuivré.

Le colorant nitré utilisé, à savoir la diméthyl-5,6 nitro-3 paraphénylènediamine, est stable dans la composition liquide.

Exemple 27

Teinture d'oxydation

On prépare la composition tinctoriale suivante :

| | |
|---|---|
| Diméthyl-2,6 nitro-3 paraphénylènediamine | 0,4 g |
| Paraphénylènediamine | 0,35 g |
| Chloro-2 paraphénylènediamine | 0,10 g |
| Paraaminophénol | 0,10 g |
| Résorcine | 0,20 g |
| Métaaminophénol | 0,30 g |
| Méthyl-2 résorcine | 0,20 g |
| Hydroquinone | 0,15 g |
| Alcool oléique glycérolé à 2 moles de glycérol | 5 g |
| Alcool oléique glycérolé à 4 moles de glycérol | 5 g |
| Acide oléique | 5 g |
| Diéthanolamide oléique | 5 g |
| Diéthanolamine oléique | 12 g |
| Alcool éthylique | 10 g |
| Ethoxy-2 éthanol | 12 g |
| Acide éthylène diaminotétracétique | 0,2 g |
| Bisulfite de sodium 35° Bé | 1,3 g |
| Ammoniaque à 22° Bé | 8 g |
| Monoéthanolamine | 3 g |
| Eau q.s.p. | 100 g |

On dilue ce liquide au moment de l'emploi avec un poids égal d'un lait oxydant titrant 20 volumes en eau oxygénée. Le gel obtenu est appliqué 30 minutes sur des cheveux châtain et on rince. On effectue un shampooing et on sèche. On obtient alors des cheveux teints dans une nuance blond foncé cuivré.

On constate que dans cette composition, le colorant nitré, à savoir la diméthyl-2,6 nitro-3 paraphénylènediamine, se conserve.

**Revendications** (pour les Etats contractants :.BE, CH, DE, FR, GB, IT, LI, NL, SE)

1. Dérivé diméthylé de nitro-3 amino-4 aniline, caractérisé par le fait qu'il répond à la formule (I) suivante :

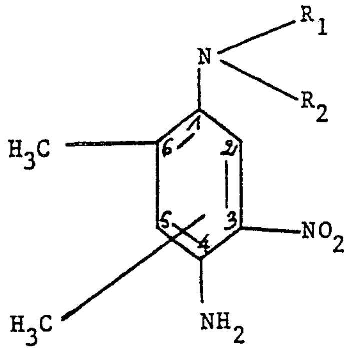

(I)

dans laquelle le radical $CH_3$ à position variable se trouve en position 2 ou 5 du noyau benzénique et $R_1$ et $R_2$ désignent, indépendamment l'un de l'autre, un atome d'hydrogène, un groupe alkyle inférieur, mono- ou poly-hydroxyalkyle inférieur ou aminoalkyle inférieur dont le groupement amino est éventuellement mono- ou disubstitué par un radical alkyle inférieur ou par un radical mono- ou polyhydroxyalkyle inférieur, étant entendu que les deux symboles $R_1$ et $R_2$ ne peuvent être simultanément différents de l'hydrogène que lorsque le radical $CH_3$ mobile se trouve en position 5, et sels cosmétiquement acceptables de ce composé.

2. Composé selon la revendication 1, caractérisé par le fait que l'un au moins des radicaux $R_1$ et $R_2$ désigne un atome d'hydrogène.

3. Composé selon la revendication 1 ou 2, caractérisé par le fait qu'il est choisi parmi le groupe comprenant : la diméthyl-2,6 nitro-3 paraphénylènediamine, la diméthyl-5,6 nitro-3 paraphénylènediamine, l'amino-4 diméthyl-2,6 nitro-3 N-méthylaniline, l'amino-4 diméthyl-5,6 nitro-3 N-β-hydroxyéthylaniline, l'amino-4 diméthyl-5,6 nitro-3 N-β,γ-dihydroxypropylaniline, l'amino-4 diméthyl-5,6 nitro-3 N-β-aminoéthylaniline, l'amino-4 diméthyl-5,6 nitro-3 N-(β-diéthylaminoéthyl) aniline.

4. Procédé de préparation d'un composé de formule (I), selon la revendication 1, dans laquelle $R_1$ et $R_2$ désignent tous deux de l'hydrogène, caractérisé par le fait que dans une première étape, on procède à la nitration du dérivé mono ou di-acétylé de formule (II) :

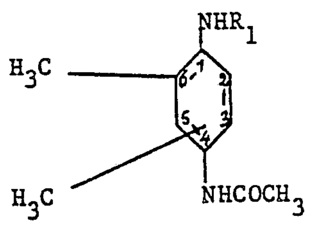

(II)

dans laquelle $R_1$ désigne un atome d'hydrogène lorsque $-CH_3$ est en position 2 et $R_1$ désigne un groupement acétyle lorsque $CH_3$ est en position 5, à l'aide d'un mélange sulfonitrique, à une température comprise entre $-5$ et 0 °C, pour obtenir le dérivé mono- ou diacétylé de formule (III) :

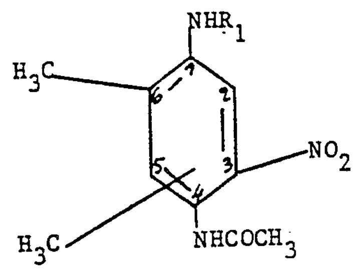

(III)

et que dans une seconde étape, on procède à l'hydrolyse du composé de formule (III) ci-dessus, à l'aide d'acide chlorhydrique.

5. Procédé selon la revendication 4, caractérisé par le fait qu'on prépare le dérivé monoacétylé de la diméthyl-2,6 paraphénylènediamine de formule (II) indiquée dans la revendication 4 dans laquelle $R_1$ désigne un atome d'hydrogène, $-CH_3$ étant en position 2, par acétylation du dichlorhydrate de diméthyl-2,6 paraphénylènediamine à l'aide d'anhydride acétique en présence d'eau et de sulfite de sodium à température ambiante.

6. Procédé de préparation d'une diméthyl-2,6 nitro-3 paraphénylène diamine de formule (I) selon la revendication 1, dans laquelle l'un des symboles $R_1$ et $R_2$ est différent de l'hydrogène, caractérisé par le

fait qu'on procède à l'alkylation d'un p-toluène sulfonamide de diméthyl-2,6 nitro-4 aniline, puis à la désulfonation de ce composé par hydrolyse, on procède ensuite à une réduction pour obtenir la diméthyl-2,6 paraphénylènediamine monosubstituée et après acétylation de l'amine primaire, on procède à la nitration et enfin à l'hydrolyse du groupement acétyle pour obtenir une diméthyl-2,6 nitro-3 paraphénylènediamine.

7. Procédé de préparation d'une diméthyl-5,6 nitro-3 paraphénylènediamine de formule (I) selon la revendication 1, dans laquelle l'un au moins des symboles $R_1$ et $R_2$ est différent de l'hydrogène, caractérisé par le fait qu'on procède directement à l'alkylation de la diméthyl-5,6 nitro-3 paraphénylènediamine à l'aide d'un halogénure d'alkyle substitué ou non, d'un époxyde ou par ouverture d'une oxazoline-1,3 one-2 intermédiaire.

8. Composition tinctoriale pour fibres kératiniques, caractérisée par le fait qu'elle contient, dans un milieu solvant, une quantité tinctorialement efficace d'au moins un composé tel que défini dans l'une quelconque des revendications 1 à 3.

9. Composition selon la revendication 8, destinée à la teinture des cheveux humains, caractérisée par le fait qu'elle contient, dans un milieu cosmétiquement acceptable, au moins un composé de formule (I) dans les proportions de 0,001 à 5 % en poids, et de préférence entre 0,05 et 2 % en poids par rapport au poids total de la composition.

10. Composition selon la revendication 8 ou 9, caractérisée par le fait qu'elle a un pH compris entre 3 et 11,5.

11. Composition tinctoriale selon la revendication 8 ou 9, caractérisée par le fait que les solvants sont choisis parmi le groupe comprenant l'eau, les alcanols inférieurs, les alcools aromatiques, les polyols, et leurs éthers ou leurs mélanges.

12. Composition selon la revendication 8 ou 9, caractérisée par le fait qu'elle contient en outre des adjuvants cosmétiques choisis parmi le groupe comprenant les agents tensio-actifs anioniques, cationiques, non-ioniques, amphotères ou leurs mélanges, les épaississants, les agents dispersants, les agents de pénétration, les séquestrants, les agents filmogènes, les tampons, les parfums, les agents alcalinisants ou acidifiants.

13. Composition selon la revendication 8 ou 9, destinée à être utilisée pour la coloration directe des cheveux humains, caractérisée par le fait qu'elle contient, en outre, d'autres colorants directs choisis parmi le groupe comprenant les colorants azoïques, anthraquinoniques et les dérivés nitrés de la série benzénique autres que ceux de formule (I).

14. Composition selon la revendication 8 ou 9, destinée à être utilisée comme lotion de mise en plis, caractérisée par le fait qu'elle se présente sous forme d'une solution aqueuse, alcoolique ou hydroalcoolique, contenant au moins une résine cosmétique.

15. Composition selon la revendication 8 ou 9, destinée à être utilisée pour la teinture d'oxydation, caractérisée par le fait qu'elle contient en outre au moins un précurseur de colorant par oxydation.

16. Composition selon la revendication 15, caractérisée parle fait qu'elle a un pH compris entre 7 et 11,5 et qu'elle contient en plus un agent réducteur.

17. Procédé de coloration des fibres kératiniques, caractérisé par le fait que l'on applique sur les fibres une quantité efficace d'une composition telle que définie dans l'une quelconque des revendications 8 à 13, on laisse poser pendant 5 à 50 minutes, et on rince, on lave éventuellement, on rince à nouveau et on sèche.

18. Procédé de coloration des fibres kératiniques, caractérisé par le fait que l'on applique, sur les fibres lavées et rincées, une quantité efficace d'une composition telle que définie dans la revendication 14, on enroule éventuellement et on sèche.

19. Procédé de coloration des fibres kératiniques, caractérisé par le fait que l'on applique sur les fibres une quantité efficace d'une composition telle que définie dans les revendications 15 ou 16, éventuellement additionnée d'un agent oxydant, on laisse poser pendant 10 à 50 minutes, on rince, on lave éventuellement au shampooing, on rince à nouveau et on sèche.

**Revendications** (pour l'Etat contractant AT)

1. Procédé de préparation d'un dérivé diméthylé de nitro-3 amino-4 aniline de formule (I) suivante :

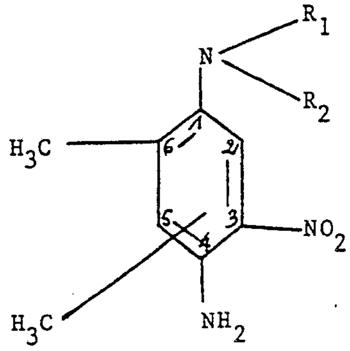

(I)

dans laquelle le radical $CH_3$ à position variable se trouve en position 2 ou 5 du noyau benzénique et $R_1$ et $R_2$ désignent tous deux un atome d'hydrogène et de sels cosmétiquement acceptables de ce composé, caractérisé par le fait que dans une première étape, on procède à la nitration du dérivé mono ou di-acétylé de formule (II) :

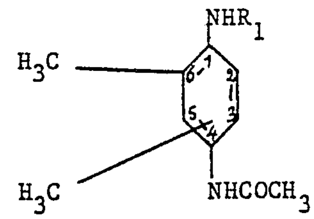

(II)

dans laquelle $R_1$ désigne un atome d'hydrogène lorsque $—CH_3$ est en position 2 et $R_1$ désigne un groupement acétyle lorsque $CH_3$ est en position 5, à l'aide d'un mélange sulfonitrique, à une température comprise entre —5 et 0 °C, pour obtenir le dérivé mono- ou diacétylé de formule (III) :

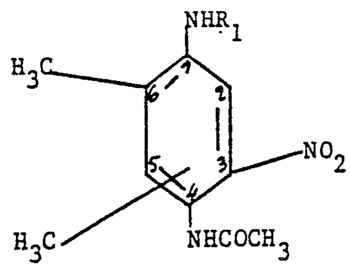

(III)

et que dans une seconde étape, on procède à l'hydrolyse du composé de formule (III) ci-dessus, à l'aide d'acide chlorhydrique.

2. Procédé selon la revendication 1, caractérisé par le fait qu'on prépare le dérivé monoacétylé de la diméthyl-2,6 paraphénylènediamine de formule (II) indiquée dans la revendication 1 dans laquelle $R_1$ désigne un atome d'hydrogène, $—CH_3$ étant en position 2, par acétylation du dichlorhydrate de diméthyl-2,6 paraphénylènediamine à l'aide d'anhydride acétique en présence d'eau et de sulfite de sodium à température ambiante.

3. Procédé selon la revendication 1 ou 2, caractérisé par le fait qu'on prépare la diméthyl-2,6 nitro-3 paraphénylènediamine ou la diméthyl-5,6, nitro-3 paraphénylènediamine.

4. Procédé de préparation d'une diméthyl-2,6 nitro-3 paraphénylènediamine de formule (I) suivante :

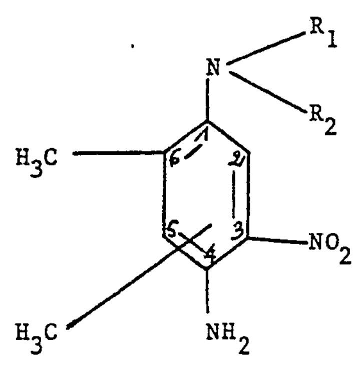

dans laquelle le radical $CH_3$ à position variable se trouve en position 2 du noyau benzénique et $R_1$ et $R_2$ désignent, indépendamment l'un de l'autre, un atome d'hydrogène, un groupe alkyle inférieur, mono- ou polyhydroxyalkyle inférieur ou aminoalkyle inférieur dont le groupement amino est éventuellement mono- ou disubstitué par un radical alkyle inférieur ou par un radical mono- ou polyhydroxyalkyle inférieur, étant entendu que l'un des deux symboles $R_1$ et $R_2$ est différent de l'hydrogène et des sels cosmétiquement acceptables de ce composé, caractérisé par le fait qu'on procède à l'alkylation d'un p-toluène sulfonamide de diméthyl-2,6 nitro-4 aniline, puis à la désulfonation de ce composé par hydrolyse, on procède ensuite à une réduction pour obtenir la diméthyl-2,6 paraphénylènediamine monosubstituée et après acétylation de l'amine primaire, on procède à la nitration et enfin à l'hydrolyse du groupement acétyle pour obtenir une diméthyl-2,6 nitro-3 paraphénylènediamine.

5. Procédé selon la revendication 4, caractérisé par le fait qu'on prépare l'amino-4 diméthyl-2,6 nitro-3 N-méthylaniline.

6. Procédé de préparation d'une diméthyl-5,6 nitro-3 paraphénylènediamine de formule (I) suivante :

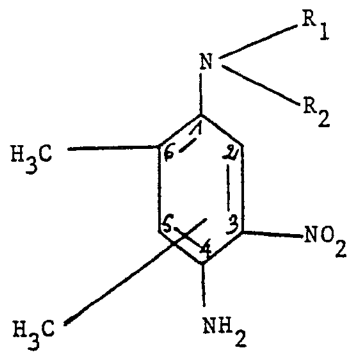

(I)

dans laquelle le radical $CH_3$ à position variable se trouve en position 5 du noyau benzénique et $R_1$ et $R_2$ désignent, indépendamment l'un de l'autre, un atome d'hydrogène, un groupe alkyle inférieur, mono-polyhydroxyalkyle inférieur ou aminoalkyle inférieur dont le groupement amino est éventuellement mono- ou disubstitué par un radical alkyle inférieur ou par un radical mono- ou polyhydroxyalkyle inférieur, étant entendu que l'un au moins des deux symboles $R_1$ et $R_2$ est différent de l'hydrogène et des sels cosmétiquement acceptables de ce composé, caractérisé par le fait qu'on procède directement à l'alkylation de la diméthyl-5,6 nitro-3 paraphénylènediamine à l'aide d'un halogénure d'alkyle substitué ou non, d'un époxyde ou par ouverture d'une oxazoline-1,3 one-2 intermédiaire.

7. Procédé selon la revendication 6, caractérisé par le fait qu'on prépare : l'amino-4 diméthyl-5,6 nitro-3 N-β-hydroxyéthylaniline, l'amino-4 diméthyl-5,6 nitro-3 N-β,γ-dihydroxypropylaniline, l'amino-4 diméthyl-5,6 nitro-3 N-β-aminoéthylaniline ou l'amino-4 diméthyl-5,6 nitro-3 N-(β-diéthylaminoéthyl) aniline.

8. Composition tinctoriale pour fibres kératiniques, caractérisée par le fait qu'elle contient, dans un milieu solvant, une quantité tinctorialement efficace d'au moins un composé préparé par le procédé décrit dans l'une quelconque des revendications 1 à 7.

9. Composition selon la revendication 8, destinée à la teinture des cheveux humains, caractérisée par le fait qu'elle contient, dans un milieu cosmétiquement acceptable, au moins un composé de formule (I) dans les proportions de 0,001 à 5 % en poids, et de préférence entre 0,05 et 2 % en poids par rapport au poids total de la composition.

10. Composition selon la revendication 8 ou 9, caractérisée par le fait qu'elle a un pH compris entre 3 et 11,5.

11. Composition tinctoriale selon la revendication 8 ou 9, caractérisée par le fait que les solvants sont choisis parmi le groupe comprenant l'eau, les alcanols inférieurs, les alcools aromatiques, les polyols, et leurs éthers ou leurs mélanges.

12. Composition selon la revendication 8 ou 9, caractérisée par le fait qu'elle contient en outre des adjuvants cosmétiques choisis parmi le groupe comprenant les agents tensio-actifs anioniques, cationiques, non ioniques, amphotères ou leurs mélanges, les épaississants, les agents dispersants, les agents de pénétration, les séquestrants, les agents filmogènes, les tampons, les parfums, les agents alcalinisants ou acidifiants.

13. Composition selon la revendication 8 ou 9, destinée à être utilisée pour la coloration directe des cheveux humains, caractérisée par le fait qu'elle contient, en outre, d'autres colorants directs choisis parmi le groupe comprenant les colorants azoïques, anthraquinoniques et les dérivés nitrés de la série benzénique autres que ceux de formule (I).

14. Composition selon la revendication 8 ou 9, destinée à être utilisée comme lotion de mise en plis, caractérisée par le fait qu'elle se présente sous forme d'une solution aqueuse, alcoolique ou hydroalcoolique, contenant au moins une résine cosmétique.

15. Composition selon la revendication 8 ou 9, destinée à être utilisée pour la teinture d'oxydation, caractérisée par le fait qu'elle contient en outre au moins un précurseur de colorant par oxydation.

16. Composition selon la revendication 15, caractérisée par le fait qu'elle a un pH compris entre 7 et 11,5 et qu'elle contient en plus un agent réducteur.

17. Procédé de coloration des fibres kératiniques, caractérisé par le fait que l'on applique sur les fibres une quantité efficace d'une composition telle que définie dans l'une quelconque des revendications 8 à 13, on laisse poser pendant 5 à 50 minutes, et on rince, on lave éventuellement, on rince à nouveau et on sèche.

18. Procédé de coloration des fibres kératiniques, caractérisé par le fait que l'on applique, sur les fibres lavées et rincées, une quantité efficace d'une composition telle que définie dans la revendication 14, on enroule éventuellement et on sèche.

19. Procédé de coloration des fibres kératiniques, caractérisé par le fait que l'on applique sur les fibres une quantité efficace d'une composition telle que définie dans les revendications 15 ou 16, éventuellement additionnée d'un agent oxydant, on laisse poser pendant 10 à 50 minutes, on rince, on lave éventuellement au shampooing, on rince à nouveau et on sèche.

**Claims** (for the Contracting States : BE, CH, DE, FR, GB, IT, LI, NL, SE)

1. Dimethylated derivative of 3-nitro-4-aminoaniline, characterized in that it corresponds to the formula (I) below :

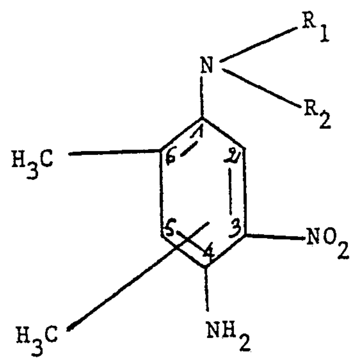

(I)

in which the $CH_3$ radical, the position of which may vary, is in position 2 or 5 of the benzene ring and $R_1$ and $R_2$ denote, independently from each other, a hydrogen atom or a lower alkyl, lower mono- or polyhydroxyalkyl or lower aminoalkyl group, the amino group of which is optionally mono- or disubstituted by a lower alkyl radical or by a lower mono- or polyhydroxyalkyl radical, it being understood that the two symbols $R_1$ and $R_2$ can simultaneously be other than hydrogen only when the movable $CH_3$ radical is in position 5, and cosmetically acceptable salts of this compound.

2. Compound according to Claim 1, characterized in that at least one of the radicals $R_1$ and $R_2$ denotes a hydrogen atom.

3. Compound according to Claim 1 or 2, characterized in that it is chosen from the group comprising : 2,6-dimethyl-3-nitro-para-phenylenediamine, 5,6-dimethyl-3-nitro-para-phenylenediamine, 4-amino-2,6-dimethyl-3-nitro-N-methylaniline, 4-amino-5,6-dimethyl-3-nitro-N-β-hydroxyethylaniline, 4-amino-5,6-dimethyl-3-nitro-N-β,γ dihydroxypropylaniline, 4-amino-5,6-dimethyl-3-nitro-N-β-aminoethylaniline, 4-amino-5,6-dimethyl-3-nitro-N-(β-diethylaminoethyl) aniline.

4. Process for the preparation of a compound of formula (I) according to Claim 1, in which $R_1$ and $R_2$ both denote hydrogen, characterized in that in a first stage, the mono- or diacetylated derivative of formula (II) :

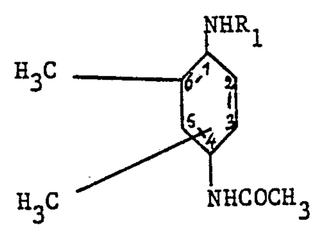

(II)

in which $R_1$ denotes a hydrogen atom when $—CH_3$ is in position 2 and $R_1$ denotes an acetyl group when $CH_3$ is in position 5, is nitrated using a sulphuric-nitric mixture, at a temperature of between —5 and 0 °C, so as to obtain the mono- or diacetylated derivative of formula (III) :

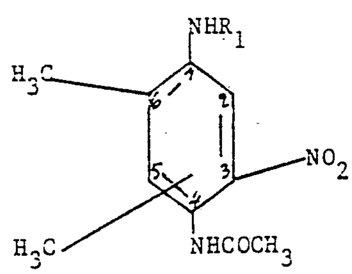

(III)

and that in a second stage, the compound of formula (III) above is hydrolyzed using hydrochloric acid.

5. Process according to Claim 4, characterized in that the monoacetylated derivative of 2,6-dimethylparaphenylenediamine of formula (II) mentioned in Claim 4, in which $R_1$ denotes a hydrogen atom, $—CH_3$ being in position 2, is prepared by the acetylation of 2,6-dimethylparaphenylenediamine hydrochloride using acetic anhydride in the presence of water and sodium sulphite at ambient temperature.

6. Process for the preparation of a 2,6-dimethyl-3-nitro-para-phenylenediamine of formula (I)

according to Claim 1, in which one of the symbols $R_1$ and $R_2$ is other than hydrogen, characterized in that a 2,6-dimethyl-4-nitroaniline p-toluenesulphonamide is alkylated and this compound is then desulphonated by hydrolysis, a reduction is then carried out in order to obtain monosubstituted 2,6-dimethylparaphenylenediamine and after the acetylation of the primary amine, the acetyl group is nitrated and finally hydrolyzed in order to obtain a 2,6-dimethyl-3-nitro-para-phenylenediamine.

7. Process for the preparation of a 5,6-dimethyl-3-nitro-para-phenylenediamine of formula (I) according to Claim 1, in which at least one of the symbols $R_1$ and $R_2$ is other than hydrogen, characterized in that 5,6-dimethyl-3-nitro-para-phenylenediamine is directly alkylated using a substituted or unsubstituted alkyl halide or an epoxide or by opening an intermediate 1,3-oxazolin-2-one.

8. Dyeing composition for keratinous fibres, characterized in that it contains, in a solvent medium, a quantity which is effective in dyeing, of at least one compound as defined in any one of Claims 1 to 3.

9. Composition according to Claim 8, intended for dyeing human hair, characterized in that it contains, in a cosmetically acceptable medium, at least one compound of formula (I) in proportions from 0.001 to 5 % by weight, and preferably between 0.05 and 2 % by weight, relative to the total weight of the composition.

10. Composition according to Claim 8 or 9, characterized in that it has a pH of between 3 and 11.5.

11. Dyeing composition according to Claim 8 or 9, characterized in that the solvents are chosen from the group comprising water, lower alkanols, aromatic alcohols, polyhydric alcohols, and their ethers or their mixtures.

12. Composition according to Claim 8 or 9, characterized in that it additionally contains cosmetic adjuvants chosen from the group comprising anionic, cationic, non-ionic or amphoteric surfactants or their mixtures, thickeners, dispersants, penetrants, sequestrants, film-forming agents, buffers, perfumes and alcalinising or acidifying agents.

13. Composition according to Claim 8 or 9, intended to be used for the direct dyeing of human hair, characterized in that it additionally contains other direct dyestuffs chosen from the group comprising azo and anthraquinone dyestuffs and nitro derivatives of the benzene series other than those of formula (I).

14. Composition according to Claim 8 or 9, intended to be used as hair setting lotion, characterized in that it is in the form of an aqueous, alcoholic or aqueous/alcoholic solution containing at least one cosmetic resin.

15. Composition according to Claim 8 or 9, intended to be used for oxidation dyeing, characterized in that it additionally contains at least one oxidation dye precursor.

16. Composition according to Claim 15, characterized in that it has a pH of between 7 and 11.5 and that it additionally contains a reducing agent.

17. Process for dyeing keratin fibres, characterized in that an effective quantity of a composition as defined in any one of Claims 8 to 13 is applied to the fibres, the fibres are exposed for 5 to 50 minutes, and rinsed, washed if required, rinsed again and dried.

18. Process for dyeing keratin fibres, characterized in that an effective quantity of a composition as defined in Claim 14 is applied to the washed and rinsed fibres, the fibres are wound on rollers and dried.

19. Process for dyeing keratin fibres, characterized in that an effective quantity of a composition as defined in Claims 15 or 16, to which an oxidizing agent has optionally been added, is applied to the fibres, the fibres are exposed for 10 to 50 minutes, rinsed, washed with shampoo if required, rinsed again and dried.

**Claims** (for the Contracting State AT)

1. Process for the preparation of a dimethylated derivative of 3-nitro-4-aminoaniline of formula (I) below :

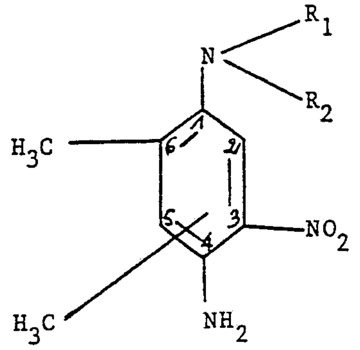

(I)

in which the radical $CH_3$, the position of which may vary, is in position 2 or 5 of the benzene ring and $R_1$ and $R_2$ both denote a hydrogen atom and of the cosmetically acceptable salts of this compound, characterized in that in a first stage, the mono-or diacetylated derivative of formula (II) :

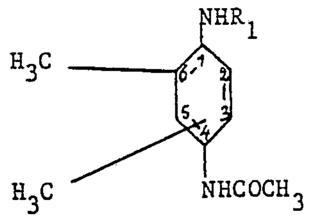

(II)

in which $R_1$ denotes a hydrogen atom when $—CH_3$ is in position 2 and $R_1$ denotes an acetyl group when $CH_3$ is in position 5, is nitrated using a sulphuric-nitric mixture, at a temperature of between —5 and 0 °C, so as to obtain the mono-or diacetylated derivative of formula (III) :

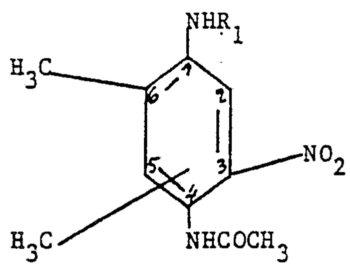

(III)

and that in a second stage, the compound of formula (III) above is hydrolyzed using hydrochloric acid.

2. Process according to Claim 1, characterized in that the monoacetylated derivative of the 2,6-dimethylparaphenylenediamine of formula (II) mentioned in Claim 1 in which $R_1$ denotes a hydrogen atom, $CH_3$ being in position 2, is prepared by the acetylation of 2,6-dimethylparaphenylenediamine dihydrochloride using acetic anhydride in the presence of water and sodium sulphite at ambient temperature.

3. Process according to Claim 1 or 2, characterized in that 2,6-dimethyl-3-nitro-para-phenylenediamine or 5,6-dimethyl-3-nitro-para-phenylenediamine is prepared.

4. Process for the preparation of a 2,6-dimethyl-3-nitro-para-phenylenediamine of formula (I) below :

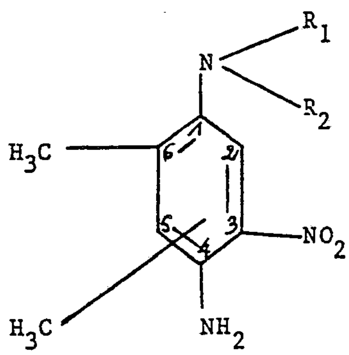

in which the radical $CH_3$, the position of which may vary, is in position 2 of the benzene ring and $R_1$ and $R_2$ denote, independently from each other, a hydrogen atom, a lower alkyl, lower mono-or polyhydroxyalkyl or lower amino alkyl group, the amino group of which is optionally mono-or disubstituted by a lower alkyl radical or by a lower mono-or polyhydroxyalkyl radical, it being understood that one of the two symbols $R_1$ and $R_2$ is other than hydrogen and of the cosmetically acceptable salts of this compound, characterized in that a 2,6-dimethyl-4-nitroaniline p-toluenesulphonamide is alkylated and this compound is desulphonated by hydrolysis, a reduction is then carried out in order to obtain monosubstituted 2,6-dimethylparaphenylenediamine and after the acetylation of the primary amine, the acetyl group is nitrated and finally hydrolyzed in order to obtain a 2,6-dimethyl-3-nitro-para-phenylenediamine.

5. Process according to Claim 4, characterized in that 4-amino-2,6-dimethyl-3-nitro-N-methylaniline is prepared.

6. Process for the preparation of a 5,6-dimethyl-3-nitro-para-phenylenediamine of formula (I) below :

(See formula (I) page 31)

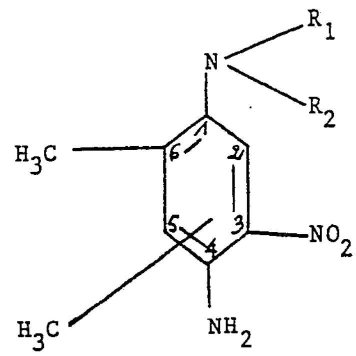

(I)

in which the radical $CH_3$, the position of which may vary, is in position 5 of the benzene ring and $R_1$ and $R_2$ denote, independently from each other, a hydrogen atom, a lower alkyl, lower mono-or polyhydroxyalkyl or lower amino alkyl group, the amino group of which may optionally be mono-or disubstituted by a lower alkyl radical or a lower mono-or polyhydroxyalkyl radical, it being understood that at least one of the two symbols $R_1$ and $R_2$ is other than hydrogen and the cosmetically acceptable salts of this compound, characterized in that 5,6-dimethyl-3-nitro-para-phenylenediamine is directly alkylated using a substituted or unsubstituted alkyl halide or an epoxide or by opening an intermediate 1,3-oxazolin-2-one.

7. Process according to Claim 6, characterized in that 4-amino-5,6-dimethyl-3-nitro-N-β-hydroxyethylaniline, 4-amino-5,6-dimethyl-3-nitro-N-β-γ-dihydroxypropylaniline, 4-amino-5,6-dimethyl-3-nitro-N-β-aminoethylaniline or 4-amino-5,6-dimethyl-3-nitro-N-(β-diethylaminoethyl) aniline is prepared.

8. Dyeing composition for keratin fibres, characterized in that it contains, in a solvent medium, a quantity which is effective in dyeing, of at least one compound prepared by the process described in any one of Claims 1 to 7.

9. Composition according to Claim 8, intended for dyeing human hair, characterized in that it contains, in a cosmetically acceptable medium, at least one compound of formula (I) in proportions from 0.001 to 5 % by weight, and preferably between 0.05 and 2 % by weight relative to the total weight of the composition.

10. Composition according to Claim 8 or 9, characterized in that it has a pH of between 3 and 11.5.

11. Dyeing composition according to Claim 8 or 9, characterized in that the solvents are chosen from the group comprising water, lower alkanols, aromatic alcohols, polyhydric alcohols, and their ethers or their mixtures.

12. Composition according to Claim 8 or 9, characterized in that it additionally contains cosmetic adjuvants chosen from the group comprising anionic, cationic, non-ionic or amphoteric surfactants or their mixtures, thickeners, dispersants, penetrants, sequestrants, film-forming agents, buffers, perfumes and alcalinising or acidifying agents.

13. Composition according to Claim 8 or 9, intended to be used for the direct dyeing of humain hair, characterized in that it additionally contains other direct dyestuffs chosen from the group comprising azo or anthraquinone dyestuffs and nitro derivatives of the benzene series other than those of formula (I).

14. Composition according to Claim 8 or 9, intended to be used as hair setting lotion, characterized in that it is in the form of an aqueous, alcoholic or aqueous/alcoholic solution containing at least one cosmetic resin.

15. Composition according to Claim 8 or 9, intended to be used for oxidation dyeing, characterized in that it additionally contains at least one oxidation dye precursor.

16. Composition according to Claim 15, characterized in that it has a pH of between 7 and 11.5 and that it additionally contains a reducing agent.

17. Process for dyeing keratin fibres, characterized in that an effective quantity of a composition as defined in any one of Claims 8 to 13 is applied to the fibres, the fibres are exposed for 5 to 50 minutes, and rinsed, washed if required, rinsed again and dried.

18. Process for dyeing keratin fibres, characterized in that an effective quantity of a composition as defined in Claim 14 is applied to the washed and rinsed fibres, the fibres are wound on a roller if required and dried.

19. Process for dyeing keratin fibres, characterized in that an effective quantity of a composition as defined in Claims 15 or 16, to which an oxidizing agent has optionally been added, is applied to the fibres, the fibres are exposed for 10 to 50 minutes, rinsed, washed with shampoo if required, rinsed again and dried.

**Patentansprüche** (für die Vertragsstaaten : BE, CH, DE, FR, GB, IT, LI, NL, SE)

1. Dimethylderivat des 3-Nitro-4-aminoanilins, dadurch gekennzeichnet, daß es der folgenden Formel (I) entspricht :

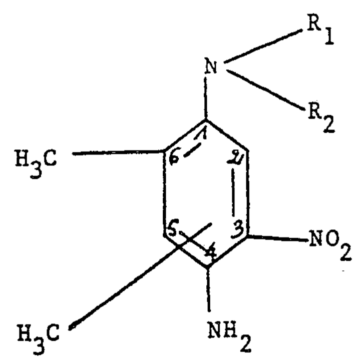

(I)

worin sich das in seiner Stellung variable Radikal $CH_3$ in der Stellung 2 oder 5 des Benzolkerns befindet und $R_1$ und $R_2$ beide unabhängig voneinander ein Wasserstoffatom, eine niedere Alkylgruppe, niedere Mono- oder Polyhydroxyalkylgruppe oder ein niederes Aminoalkyl bedeutet, dessen Aminogruppe gegebenenfalls durch ein niederes Alkylradikal oder durch ein niederes Mono- oder Polyhydroxyalkylradikal mono- oder disubstituiert ist, wobei die beiden Reste $R_1$ und $R_2$ nicht gleichzeitig verschieden von Wasserstoff sein können, wenn das mobile Radikal $CH_3$ sich in Stellung 5 befindet, und kosmetisch akzeptable Salze dieser Verbindung.

2. Verbindung gemäß Anspruch 1, dadurch gekennzeichnet, daß zumindest eines der Radikale $R_1$ und $R_2$ ein Wasserstoffatom ist.

3. Verbindung nach Anspruch 1 oder 2, dadurch gekennzeichnet, daß sie aus der folgenden Gruppe ausgewählt ist: 2,6-Dimethyl-3-nitroparaphenylendiamin, 5,6-Dimethyl-3-nitroparaphenylendiamin, 4-Amino-2,6-dimethyl-3-nitro-N-methylanilin, 4-Amino-5,6-dimethyl-3-nitro-N-β-hydroxyäthylanilin, 4-Amino-5,6-dimethyl-3-nitro-N-β-γ-dihydroxypropylanilin, 4-Amino-5,6-dimethyl-3-nitro-N-β-aminoäthylanilin und 4-Amino-5,6-dimethyl-3-nitro-N-(β-diäthylaminoäthyl) anilin.

4. Verfahren zur Herstellung einer Verbindung der Formel (I) gemäß Anspruch 1, wobei $R_1$ und $R_2$ beide Wasserstoff bedeuten, dadurch gekennzeichnet, daß man in einer ersten Stufe mit Hilfe einer Nitriersäure die Nitrierung des Mono- oder Diacetylderivates der Formel (II):

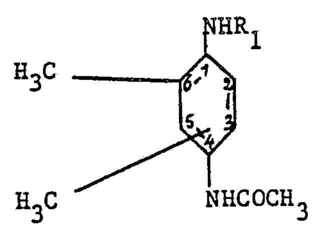

(II)

in der $R_1$ ein Wasserstoffatom bezeichnet, wenn $CH_3$ in Stellung 2 ist und $R_1$ eine Acetylgruppe bezeichnet, wenn $CH_3$ in Stellung 5 ist, bei einer Temperatur zwischen —5 und 0 °C durchführt, um das Mono- oder Diacetylderivat der Formel (III) zu erhalten:

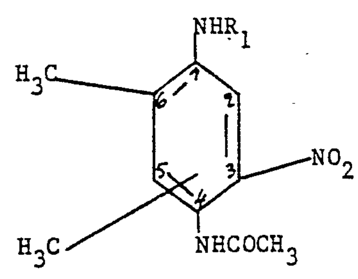

(III)

und daß in einer zweiten Stufe die Verbindung der obengenannten Formel (III) mit Hilfe von Salzsäure hydrolysiert wird.

5. Verfahren nach Anspruch 4, dadurch gekennzeichnet, daß man das Monoacetylderivat des 2,6-Dimethyl-paraphenylendiamins der Formel (II) gemäß Anspruch 4, in der $R_1$ ein Wasserstoffatom ist und $—CH_3$ in Stellung 2 steht, durch Acetylierung des Dichlorhydrates des 2,6-Dimethylparaphenylendiamins mit Hilfe von Essigsäureanhydrid in Gegenwart von Wasser und Natriumsulfit bei Raumtemperatur herstellt.

6. Verfahren zur Herstellung von 2,6-Dimethyl-3-nitroparaphenylendiamin der Formel (I) gemäß Anspruch 1, wobei einer der Reste $R_1$ und $R_2$ verschieden von Wasserstoff ist, dadurch gekennzeichnet, daß man die Alkylierung eines 2,6-Dimethyl-4-nitroanilin-p-toluylsulfonamids durchführt, darauf mittels

Hydrolyse diese Verbindung desulfoniert, weiters eine Reduktion durchführt, um das monosubstituierte 2,6-Dimethylparaphenylendiamin zu erhalten, nach Acetylierung des primären Amins die Nitrierung durchführt und endlich die Acetylgruppe hydrolysiert, um ein 2,6-Dimethyl-3-nitroparaphenylendiamin zu erhalten.

7. Verfahren zur Herstellung eines 5,6-Dimethyl-3-nitroparaphenylendiamins der Formel (I) gemäß Anspruch 1, worin zumindest einer der Reste $R_1$ und $R_2$ verschieden von Wasserstoff ist, dadurch gekennzeichnet, daß man direkt die Alkylierung des 5,6-Dimethyl-3-nitroparaphenylendiamins mit Hilfe eines substituierten oder nicht-substituierten Alkylhalogenids, eines Epoxyds oder durch Öffnung eines 1,3-Oxazolin-2-on-Zwischenproduktes durchführt.

8. Färbemittel für Keratinfasern, dadurch gekennzeichnet, daß es in einem Lösungsmittel in einer färbewirksamen Menge zumindest eine Verbindung gemäß einem der vorhergehenden Ansprüche 1 bis 3 enthält.

9. Mittel nach Anspruch 8 für die Färbung menschlicher Haare, dadurch gekennzeichnet, daß es in einem kosmetisch akzeptablen Milieu zumindest eine Verbindung der Formel (I) in den Mengen zwischen 0,001 und 5 Gew.-%, und in bevorzugter Weise zwischen 0,05 und 2 Gew.-%, bezogen auf das Gesamtgewicht des Mittels, enthält.

10. Mittel nach Anspruch 8 oder 9, dadurch gekennzeichnet, daß es einen pH-Wert zwischen 3 und 11,5 aufweist.

11. Färbemittel nach Anspruch 8 oder 9, dadurch gekennzeichnet, daß die Lösungsmittel aus der Gruppe Wasser, niedere Alkanole, aromatische Alkohole, Polyole, deren Äther oder deren Mischungen gewählt sind.

12. Mittel nach Anspruch 8 oder 9, dadurch gekennzeichnet, daß es weiters kosmetische Adjuvantien enthält, die aus der Gruppe anionische, kationische, nichtionische oder amphotere oberflächenaktive Mittel oder deren Mischungen, Verdickungsmittel, Dispergierungsmittel, Penetrierungsmittel, Sequestrierungsmittel, filmbildende Mittel, Puffer, Parfums, Alkalisierungs- oder Ansäuerungsmittel besteht.

13. Mittel nach Anspruch 8 oder 9 zur Direktfärbung menschlicher Haare, dadurch gekennzeichnet, daß es weiters andere Direktfarbstoffe enthält, die aus der Gruppe Azofarbstoffe, Antrachinonfarbstoffe und den Nitroderivaten der Benzolreihe besteht, die von jenen der Formel (I) verschieden sind.

14. Mittel nach Anspruch 8 oder 9 zur Verwendung als Wasserwellenlotion dadurch gekennzeichnet, daß es in Form einer wäßrigen, alkoholischen oder wasser-alkoholischen Lösung vorliegt und zumindest ein kosmetisches Harz enthält.

15. Mittel nach Anspruch 8 oder 9 für die Oxidationsfärbung, dadurch gekennzeichnet, daß es weiters zumindest einen Oxidationsfarbstoffvorläufer enthält.

16. Mittel nach Anspruch 15, dadurch gekennzeichnet, daß es einen pH-Wert zwischen 7 und 11,5 aufweist und zusätzlich ein Reduktionsmittel enthält.

17. Färbeverfahren für Keratinfasern, dadurch gekennzeichnet, daß man auf die Fasern eine wirksame Menge des Mittels nach einem der Ansprüche 8 bis 13 aufträgt, während 5 bis 50 Minuten aufgetragen läßt, spült, gegebenenfalls wäscht, neuerlich spült und trocknet.

18. Färbeverfahren für Keratinfasern, dadurch gekennzeichnet, daß man auf die gewaschenen und gespülten Fasern eine wirksame Menge eines Mittels gemäß Anspruch 14 aufträgt, gegebenenfalls aufwickelt und trocknet.

19. Färbeverfahren für Keratinfasern, dadurch gekennzeichnet, daß man auf die Fasern eine wirksame Menge eines Mittel gemäß den Ansprüchen 15 oder 16 aufträgt, dem gegebenenfalls ein Oxidationsmittel zugegeben wurde, während 10 bis 50 Minuten aufgetragen läßt, spült, gegebenenfalls wäscht und shampooniert, neuerlich spült und trocknet.

**Patentansprüche** (für den Vertragsstaat AT)

1. Verfahren zur Herstellung eines Dimethylderivates des 3-Nitro-4-aminoanilins der folgenden Formel (I) :

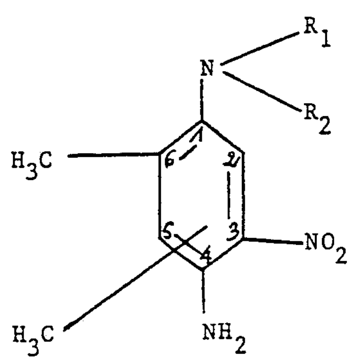

(I)

worin sich das in seiner Stellung variable Radikal $CH_3$ in der Stellung 2 oder 5 des Benzolkerns befindet und $R_1$ und $R_2$ beide ein Wasserstoffatom bezeichnen, sowie der kosmetisch akzeptablen Salze dieser Verbindung, dadurch gekennzeichnet, daß man in einer ersten Stufe mit Hilfe einer Nitriersäure die Nitrierung des Mono- oder Diacetylderivates der Formel (II) :

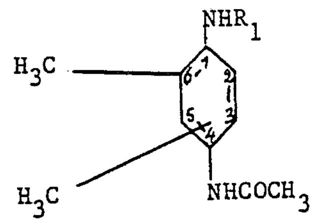

(II)

in der $R_1$ ein Wasserstoffatom bezeichnet, wenn $CH_3$ in Stellung 2 ist und $R_1$ eine Acetylgruppe bezeichnet, wenn $CH_3$ in Stellung 5 ist, bei einer Temperatur zwischen —5 und 0 °C durchführt, um das Mono- oder Diacetylderivat der Formel (III) zu erhalten :

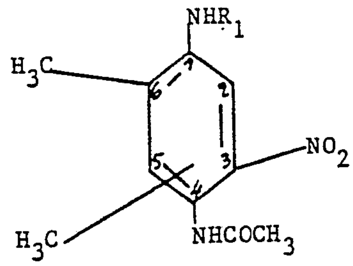

(III)

und daß in einer zweiten Stufe die Verbindung der obengenannten Formel (III) mit Hilfe von Salzsäure hydrolysiert wird.

2. Verfahren nach Anspruch 1, dadurch gekennzeichnet, daß man das Monoacetylderivat des 2,6-Dimethyl-paraphenylendiamins der Formel (II) gemäß Anspruch 1, in der $R_1$ ein Wasserstoffatom ist und $-CH_3$ in Stellung 2 steht, durch Acetylierung des Dichlorhydrates des 2,6-Dimethylparaphenylendiaminis mit Hilfe von Essigsäureanhydrid in Gegenwart von Wasser und Natriumsulfit bei Raumtemperatur herstellt.

3. Verfahren nach Anspruch 1 oder 2, dadurch gekennzeichnet, daß man das 2,6-Dimethyl-3-nitroparaphenylendiamin oder das 5,6-Dimethyl-3-nitroparaphenylendiamin herstellt.

4. Verfahren zur Herstellung von 2,6-Dimethyl-3-nitroparaphenylendiamin der folgenden Formel (I) :

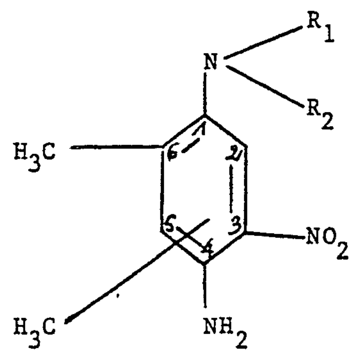

worin das in seiner Stellung variable Radikal $CH_3$ sich in Stellung 2 des Benzolkerns befindet und $R_1$ und $R_2$ unabhängig voneinander ein Wasserstoffatom, eine niedere Alkylgruppe, niedere Mono- oder Polyhydroxyalkylgruppe oder ein niederes Aminoalkyl bedeutet, dessen Aminogruppe gegebenenfalls durch ein niederes Alkylradikal oder durch ein niederes Mono- oder Polyhydroxyalkylradikal mono- oder disubstituiert ist, wobei einer der beiden Reste $R_1$ und $R_2$ verschieben von Wasserstoff ist, und kosmetisch akzeptabler Salze dieser Verbindung, dadurch gekennzeichnet, daß man die Alkylierung eines 2,6-Dimethyl-4-nitroanilin-p-toluylsulfonamids durchführt, darauf mittels Hydrolyse diese Verbindung desulfoniert, weiters eine Reduktion durchführt, um das monosubstituierte 2,6-Dimethylparaphenylendiamin zu erhalten, nach Acetylierung des primären Amins die Nitrierung durchführt und endlich die Acetylgruppe hydrolysiert, um ein 2,6-Dimethyl-3-nitroparaphenylendiamin zu erhalten.

5. Verfahren nach Anspruch 4, dadurch gekennzeichnet, daß man das 4-Amino-2,6-dimethyl-3-nitro-N-methylanilin herstellt.

6. Verfahren zur Herstellung eines 5,6-Dimethyl-3-nitroparaphenylendiamins der folgenden Formel (I)

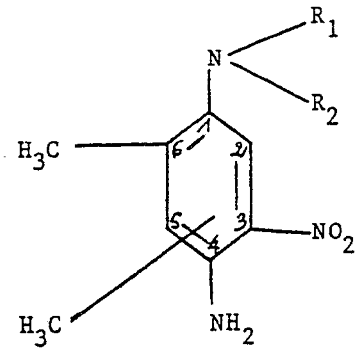

(I)

worin das sich in variabler Stellung befindliche Radikal $CH_3$ in Stellung 5 des Benzolkerns befindet und $R_1$ und $R_2$ unabhängig voneinander Wasserstoffatom, niedere Alkylgruppe, niederes Mono- oder Polyhydroxyalkyl oder niederes Aminoalkyl bedeuten, dessen Aminogruppe gegebenenfalls durch ein niederes Alkylradial oder durch ein niederes Mono- oder Polyhydroxyalkylradikal mono- oder disubstituiert ist, wobei zumindest einer der beiden Reste $R_1$ oder $R_2$ verschieden von Wasserstoff ist, sowie von kosmetisch akzeptablen Salzen dieser Verbindung, dadurch gekennzeichnet, daß man direkt die Alkylierung des 5,6-Dimethyl-3-nitroparaphenylendiamins mit Hilfe eines substituierten oder nichtsubstituierten Alkylhalogenids, eines Epoxyds oder durch Öffnung eines 1,3-Oxazolin-2-on-Zwischenproduktes durchführt.

7. Verfahren nach Anspruch 6, dadurch gekennzeichnet, daß man folgende Verbindungen herstellt : 4-Amino-5,6-dimethyl-3-nitro-N-β-hydroxyäthylanilin, 4-Amino-5,6-dimethyl-3-nitro-N-β,γ-dihydroxypropylanilin, 4-Amino-5,6-dimethyl-3-nitro-N-β-aminoäthylanilin oder 4-Amino-5,6-dimethyl-3-nitro-N-(β-diäthylaminoäthyl) anilin.

8. Färbemittel für Keratinfasern, dadurch gekennzeichnet, daß es in einem Lösungsmittel in einer färbewirksamen Menge zumindest eine Verbindung enthält, die gemäß einem der vorhergehenden Ansprüche 1 bis 7 hergestellt ist.

9. Mittel nach Anspruch 8 für die Färbung menschlicher Haare, dadurch gekennzeichnet, daß es in einem kosmetisch akzeptablen Milieu zumindest eine Verbindung der Formel (I) in den Mengen zwischen 0,001 und 5 Gew.-%, und in bevorzugter Weise zwischen 0,05 und 2 Gew.-%, bezogen auf das Gesamtgewicht des Mittels, enthält.

10. Mittel nach Anspruch 8 oder 9, dadurch gekennzeichnet, daß es einen pH-Wert zwischen 3 und 11,5 aufweist.

11. Färbemittel nach Anspruch 8 oder 9, dadurch gekennzeichnet, daß die Lösungsmittel aus der Gruppe Wasser, niedere Alkanole, aromatische Alkohole, Polyole, deren Äther oder deren Mischungen gewählt sind.

12. Mittel nach Anspruch 8 oder 9, dadurch gekennzeichnet, daß es weiters kosmetische Adjuvantien enthält, die aus der Gruppe anionische, kationische, nichtionische oder amphotere oberflächenaktive Mittel oder deren Mischungen, Verdickungsmittel, Dispergierungsmittel, Penetrierungsmittel, Sequestrierungsmittel, filmbildende Mittel, Puffer, Parfums, Alkalisierungs- oder Ansäuerungsmittel besteht.

13. Mittel nach Anspruch 8 oder 9 zur Direktfärbung menschlicher Haare, dadurch gekennzeichnet, daß es weiters andere Direktfarbstoffe enthält, die aus der Gruppe Azofarbstoffe, Antrachinonfarbstoffe und den Nitroderivaten der Benzolreihe besteht, die von jenen der Formel (I) verschieden sind.

14. Mittel nach Anspruch 8 oder 9 zur Verwendung als Wasserwellenlotion dadurch gekennzeichnet, daß es in Form einer wäßrigen, alkoholischen oder wasser-alkoholischen Lösung vorliegt und zumindest ein kosmetisches Harz enthält.

15. Mittel nach Anspruch 8 oder 9 für die Oxidationsfärbung, dadurch gekennzeichnet, daß es weiters zumindest einen Oxidationsfarbstoffvorläufer enthält.

16. Mittel nach Anspruch 15, dadurch gekennzeichnet, daß es einen pH-Wert zwischen 7 und 11,5 aufweist und zusätzlich ein Reduktionsmittel enthält.

17. Färbeverfahren für Keratinfasern, dadurch gekennzeichnet, daß man auf die Fasern eine wirksame Menge des Mittels nach einem der Ansprüche 8 bis 13 aufträgt, während 5 bis 50 Minuten aufgetragen läßt, spült, gegebenenfalls wäscht, neuerlich spült und trocknet.

18. Färbeverfahren für Keratinfasern, dadurch gekennzeichnet, daß man auf die gewaschenen und gespülten Fasern eine wirksame Menge eines Mittels gemäß Anspruch 14 aufträgt, gegebenenfalls aufwickelt und trocknet.

19. Färbeverfahren für Keratinfasern, dadurch gekennzeichnet, daß man auf die Fasern eine wirksame Menge eines Mittels gemäß den Ansprüchen 15 oder 16 aufträgt, dem gegebenenfalls ein Oxidationsmittel zugegeben wurde, während 10 bis 50 Minuten aufgetragen läßt, spült, gegebenenfalls wäscht und shampooniert, neuerlich spült und trocknet.